# EUROPEAN PATENT APPLICATION

(11) **EP 4 174 065 A1**
(43) Date of publication of application: **03.05.2023**
(21) Application number: 21832469.7
(22) Date of filing: 29.06.2021
(51) Int. Cl.: C07D 417/14, A61K 31/506, A61P 25/00, A61P 25/14, A61P 25/16, A61P 25/18, A61P 25/22, A61P 25/24, A61P 25/28, A61P 25/30

(54) **SALT OF 2-(SUBSTITUTED PYRIMIDINYL)THIAZOLECARBOXAMIDE COMPOUND, AND COMPOSITION AND USE THEREOF**

(30) Priority: 30.06.2020 CN 202010612905
(71) Applicant: Sunshine Lake Pharma Co., Ltd., Dongguan, Guangdong 523000 (CN)
(72) Inventor: JIN, Chuanfei, Dongguan, Guangdong 523871 (CN); XU, Tengfei, Dongguan, Guangdong 523871 (CN); YE, Huiqing, Dongguan, Guangdong 523871 (CN)
(74) Representative: Hoffmann Eitle
(86) International application number: PCT/CN2021/102949
(87) International publication number: WO 2022/002011

(57) **Abstract**

The present invention relates to a salt of a 2-(substituted pyrimidinyl)thiazole carboxamide compound, and a composition and use thereof, and also relates to a crystal form of the salt. Specifically, the present invention relates to a pharmaceutical composition comprising the salt, and a use of the salt or pharmaceutical composition in the preparation of a drug for preventing, treating or alleviating central nervous system dysfunction, particularly depression.

## Description

### FIELD OF THE INVENTION

The invention belongs to the technical field of medicine, and relates to salts of 2-(substituted pyrimidinyl) thiazole carboxamide compounds, compositions and uses thereof. It specifically relates to salts of 2-(2-(4-(3-(5-cyano-1*H*-indol-3-yl)propyl)piperazin-1-yl)pyrimidin-5-yl)-4-methylthiazol-5-carboxamide, crystal forms of the salts and uses thereof, and further relates to pharmaceutical compositions comprising the salts or crystal forms and uses thereof.

### BACKGROUND ART

5-Hydroxytryptamine, a neurotransmitter that transmits signals in the brain and nervous system, plays an important role in central nervous system (CNS) dysfunction, especially in anxiety, depression, aggression and impulsive emotions. Antagonism or activation of certain 5-hydroxytryptamine receptors can effectively regulate central nervous system dysfunction. 5-HT_{1A} receptor, a G-protein coupled receptor, is widely distributed in regions that can receive 5-hydroxytryptamine from raphe nucleus, including frontal cortex, lateral septum, amygdala, hippocampus and hypothalamus. In these cortical marginal regions, 5-HT_{1A} is located in the postsynaptic membrane. At the same time, 5-HT_{1A} receptor is also the presynaptic membrane autoreceptor on the raphe nucleus, which can reduce the firing rate of neurons (i.e., the amount of 5-hydroxytryptamine released per action potential), and the synthesis of neurotransmitters, thereby reducing the activity of 5-hydroxytryptamine in the projection area. Activating the 5-HT_{1A} receptor of the presynaptic membrane can inhibit the synthesis of tyrosine hydroxylase and the activity of glutamate channel (produced in the medial prefrontal cortex, pointing to the raphe nucleus), thereby indirectly reducing the transport of 5-hydroxytryptamine (Jonathan Savitz, Irwin Lucki, Wayne C. Dreams. 5-HT1A receiver function in major depression disorder. Prog Neurobiol. 2009, 88 (1): 17-31).

Among all indications related to 5-hydroxytryptamine dysfunction, depression is the most harmful to human health. According to the report of Depression and Other Common Mental Disorders released by the World Health Organization (WHO) in 2017, it is estimated that more than 300 million people worldwide are suffering from depression, and the global average incidence rate is about 4.4%.

Traditional selective 5-hydroxytryptamine reuptake inhibitors SSRIs treat depression by inhibiting 5-hydroxytryptamine reuptake and regulating its transport to increase the content of 5-hydroxytryptamine. But after the use of SSRIs, the 5-HT_{1A} autoreceptor in the presynaptic membrane is also activated, resulting in a decrease in the release of 5-hydroxytryptamine, and a decrease in the concentration of 5-hydroxytryptamine between synapses. However, with the prolongation of administration time, SSRIs can desensitize the 5-HT_{1A} autoreceptor, suppress the activation effect, and play a normal regulatory role. It is inferred that the activation effect on 5-HT_{1A} autoreceptor is an important reason for delaying the efficacy of SSRIs (Celada P, Puig M, Amargos-Bosch M, et al., The therapeutic role of 5-HT1A and 5-HT2A receptors in depression. J Psychiatry Neurosci, 2004, 29(4): 252-65). Therefore, overcoming the negative feedback effects of 5-HT_{1A} autoreceptor antagonists has the prospect of enhancing and accelerating clinical antidepressants.

Compared with SSRIs, 5-HT_{1A} receptor agonists or partial agonists act directly on postsynaptic 5-hydroxytryptamine receptors to increase 5-hydroxytryptamine neurotransmission during SSRI latency. Feiger and Wilcox demonstrated that buspirone and gepirone are clinically effective partial agonists of 5-HT_{1A} (Feiger, A. Psychopharmacol. Bull. 1996, 32: 659-65). The addition of buspirone to standard SSRI therapy resulted in significant improvement in patients who had previously failed to respond to standard therapy for depression (Dimitriou, E.J. Clin. Psychopharmacol., 1998, 18: 465-9).

Different salts or different solid forms of the active pharmaceutical ingredient may have different properties. Different salts and/or solid forms may have significant differences in appearance, solubility, melting point, dissolution, bioavailability, *etc.,* and may have different effects on the stability, bioavailability and efficacy. Therefore, the drug should be fully considered the problem of salts and/or solid forms in drug research and development.

International application WO 2019062662 A1 discloses the compound 2-(2-(4-(3-(5-cyano-1*H*-indol-3-yl)propyl)piperazin-1-yl)pyrimidin-5-yl)-4-methylthiazol-5-carboxamide (the compound having formula (I)), which has selective 5-hydroxytryptamine reuptake inhibitory activity and/or 5-HT_{1A} receptor agonistic activity, and has potential antidepressant effects. However, the prior art has not disclosed any salt or crystal form of the compound.

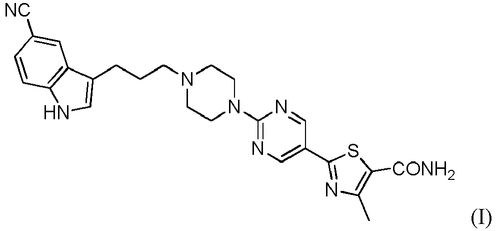

### SUMMARY

Through a large number of experimental studies, the inventor found that after the compound having formula (I) is formed into a salt with a suitable acid, the preparation purity of the product is significantly improved, and the physical properties and various properties are more suitable for industrial production, and more conducive to formulation development. The inventors have studied the salt formation process of the compound having the formula (I) and different acids, the solid form of the obtained salt, its physicochemical properties and its pharmacological properties, and found that the compound does not necessarily form stable salts when reacted with different acids. For example, the compound of formula (I) reacts with acids commonly used in the art such as maleic acid, citric acid, benzoic acid, *etc.* and cannot form a salt, but the compound of formula (I) can be combined into salts with phosphoric acid, sulfuric acid, p-toluenesulfonic acid, benzenesulfonic acid and L-tartaric acid. Further research found that the phosphate obtained by combining the compound with phosphoric acid has stable properties and can be greatly improved in all aspects, especially the phosphate crystal form A. Compared with other salts or other crystal forms of phosphate, its pharmacokinetics, stability and other properties are better. Therefore, the phosphate crystal form A of the compound having formula (I) of the present invention has better properties, better pharmacokinetic properties, and thus better druggability.

Specifically, the present invention relates to salts of compound having formula (I), and the use of the salts of the compound or pharmaceutical compositions comprising the salts in the manufacture of a medicament for preventing, treating or alleviating central nervous system dysfunction, especially depression. The salt described in the present invention is phosphates and the like. Further, the salt of the present invention is phosphate crystal form A. The crystal form of the present invention may also be in the form of a solvate, such as a hydrate form, or a DMF solvate form.

In one aspect, the present invention provides a salt of a compound having formula (I),

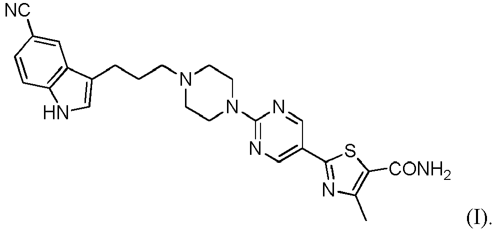

In some embodiments, the salt described herein is phosphate, tartrate (e.g., L-tartrate), benzenesulfonate,p-toluenesulfonate, or sulfate.

In some embodiments, the salt of the compound having formula (I) described in the present invention is phosphate.

In some embodiments, the salt of the present invention is a phosphate of the compound having formula (I), wherein the phosphate is phosphate crystal form A1, phosphate crystal form A, phosphate crystal form B, phosphate crystal form C, phosphate crystal form G or phosphate crystal form H.

In other embodiments, the X-ray powder diffraction pattern of the phosphate crystal form A of the present invention comprises peaks expressed as 2Θ at: 11.14°±0.2°, 12.25°±0.2°, 13.50°±0.2°, 15.98°± 0.2°, 16.38°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 11.14°±0.2°, 12.25°±0.2°, 13.50°±0.2°, 15.98°± 0.2°, 16.28°± 0.2, 16.38°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 9.65°± 0.2°, 11.14°±0.2°, 12.25°±0.2°, 13.50°±0.2°, 15.98°± 0.2°, 16.28°± 0.2, 16.38°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 11.14°± 0.2°, 12.25°±0.2°, 13.50°± 0.2°, 15.98°± 0.2°, 16.38°± 0.2°, 19.36°±0.2°, 20.31°± 0.2°, 20.79°± 0.2, 20.93°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 11.14°± 0.2°, 12.25°±0.2°, 13.50°± 0.2°, 15.98°± 0.2°, 16.28°± 0.2°, 16.38°± 0.2°, 19.36°±0.2°, 20.31°± 0.2°, 20.79°± 0.2, 20.93°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 11.14°± 0.2°, 12.25°±0.2°, 13.50°± 0.2°, 15.98°± 0.2°, 16.28°± 0.2°, 16.38°± 0.2°, 19.36°±0.2°, 20.31°± 0.2°, 20.79°± 0.2, 20.93°± 0.2°, 21.68°± 0.2°, 22.52°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 9.65°± 0.2°, 11.14°±0.2°, 12.25°± 0.2°, 13.50°± 0.2°, 15.98°± 0.2°, 16.28°± 0.2°, 16.38°±0.2°, 19.36°± 0.2°, 20.31°± 0.2, 20.79°± 0.2°, 20.93°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 9.65°± 0.2°, 11.14°± 0.2°, 12.25°±0.2°, 13.50°± 0.2°, 15.98°± 0.2°, 16.28°± 0.2°, 16.38°± 0.2°, 19.36°±0.2°, 20.31°± 0.2°, 20.79°± 0.2, 20.93°± 0.2°, 21.68°± 0.2°, 22.52°± 0.2°, 25.26°± 0.2°, 25.98°± 0.2°, 26.82°± 0.2°.

In other embodiments, the X-ray powder diffraction pattern of the phosphate crystal form A of the present invention comprises peaks expressed as 2Θ at: 9.65°± 0.2°, 11.14°± 0.2°, 12.25°±0.2°, 13.50°± 0.2°, 15.98°± 0.2°, 16.28°± 0.2°, 16.38°± 0.2°, 19.36°±0.2°, 20.31°± 0.2°, 20.79°± 0.2, 20.93°± 0.2°, 21.68°± 0.2°, 22.52°±0.2°, 25.26°±0.2°, 25.98°± 0.2°, 26.82°± 0.2°, 36.35°± 0.2°.

In other embodiments, the X-ray powder diffraction pattern of the phosphate crystal form A of the present invention comprises peaks expressed as 2Θ at: 9.65°± 0.2°, 11.14°± 0.2°, 12.25°± 0.2°, 13.50°± 0.2°, 15.98°± 0.2°, 16.38°± 0.2°, 16.86°±0.2°, 17.20°±0.2°, 19.36°± 0.2°, 20.31°± 0.2°, 20.79°± 0.2°, 20.93°± 0.2°, 21.68°±0.2°, 22.52°±0.2°, 24.73°±0.2°, 25.26°±0.2°, 25.98°± 0.2°, 26.82°± 0.2°, 29.82°±0.2°, 30.58°± 0.2°, 31.43°± 0.2°, 32.14°± 0.2°.

In other embodiments, the X-ray powder diffraction pattern of the phosphate crystal form A of the present invention comprises peaks expressed as 2Θ at: 9.65°± 0.2°, 11.14°± 0.2°, 12.25°± 0.2°, 13.50°± 0.2°, 15.98°± 0.2°, 16.28°± 0.2°, 16.38°± 0.2°, 16.86°±0.2°, 19.36°± 0.2°, 20.31°± 0.2°, 20.79°± 0.2°, 20.93°± 0.2°, 21.68°± 0.2°, 22.52°± 0.2°, 24.73°± 0.2°, 25.26°±0.2°, 25.98°± 0.2°, 26.82°± 0.2°, 29.82°± 0.2°, 30.58°± 0.2°, 31.43°± 0.2°, 32.14°± 0.2°.

In other embodiments, the X-ray powder diffraction pattern of the phosphate crystal form A of the present invention comprises peaks expressed as 2Θ at: 9.65°± 0.2°, 11.14°± 0.2°, 12.25°± 0.2°, 13.50°± 0.2°, 15.98°± 0.2°, 16.28°± 0.2°, 16.38°± 0.2°, 16.86°±0.2°, 17.20°±0.2°, 19.36°± 0.2°, 20.31°± 0.2°, 20.79°± 0.2°, 20.93°± 0.2°, 21.68°± 0.2°, 22.52°± 0.2°, 24.73°± 0.2°, 25.26°± 0.2°, 25.98°± 0.2°, 26.82°± 0.2°, 29.82°± 0.2°, 30.58°± 0.2°, 31.43°± 0.2°, 32.14°± 0.2°.

In other embodiments, the X-ray powder diffraction pattern of the phosphate crystal form A of the present invention comprises peaks expressed as 2Θ at: 9.65°± 0.2°, 11.14°± 0.2°, 12.25°± 0.2°, 13.50°± 0.2°, 15.98°± 0.2°, 16.28°± 0.2°, 16.38°± 0.2°, 16.86°±0.2°, 17.20°±0.2°, 19.36°± 0.2°, 20.31°± 0.2°, 20.79°± 0.2°, 20.93°± 0.2°, 21.68°± 0.2°, 22.52°± 0.2°, 24.73°± 0.2°, 25.26°± 0.2°, 25.98°± 0.2°, 26.82°± 0.2°, 29.82°± 0.2°, 30.58°± 0.2°, 31.43°± 0.2°, 32.14°± 0.2°, 36.35°± 0.2°, 36.87°± 0.2°.

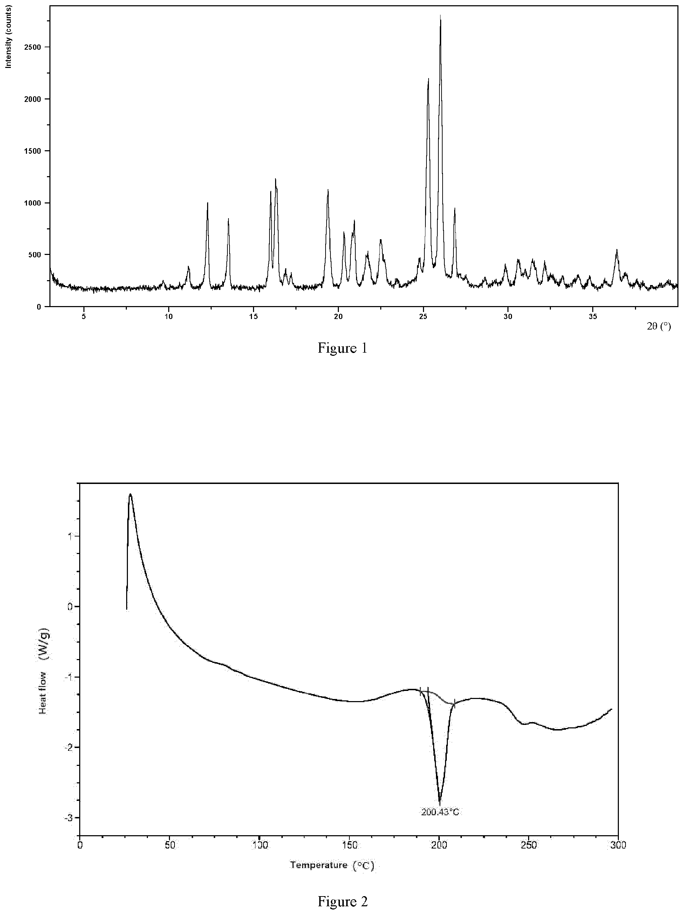

In yet other embodiments, the phosphate crystal form A of the present invention has an X-ray powder diffraction pattern substantially as shown in Figure 1.

In other embodiments, the differential scanning calorimetry diagram of the phosphate crystal form A of the present invention comprises an endothermic peak at 200.43°C ± 3°C.

In yet other embodiments, the phosphate crystal form A of the present invention has a differential scanning calorimetry diagram substantially as shown in Figure 2.

In other embodiments, the X-ray powder diffraction pattern of the phosphate crystal form B of the present invention comprises peaks expressed as 2Θ at: 8.17°±0.2°, 10.08°±0.2°, 12.38°±0.2°, 17.77°± 0.2°, 19.18°± 0.2, 20.52°± 0.2°, 23.67°± 0.2°, 25.89°± 0.2°, 26.39°± 0.2°.

In other embodiments, the X-ray powder diffraction pattern of the phosphate crystal form B of the present invention comprises peaks expressed as 2θ at: 8.17°± 0.2°, 10.08°±0.2°, 12.38°± 0.2°, 16.55°± 0.2°, 17.77°± 0.2°, 19.18°±0.2°, 20.52°± 0.2°, 23.67°± 0.2, 25.89°± 0.2°, 26.39°± 0.2°, 28.13°± 0.2°, 28.91°± 0.2°.

In other embodiments, the X-ray powder diffraction pattern of the phosphate crystal form B of the present invention comprises peaks expressed as 2Θ at: 8.17°± 0.2°, 10.08°±0.2°, 12.38°± 0.2°, 14.82°± 0.2°, 16.55°± 0.2°, 17.77°± 0.2°, 19.18°±0.2°, 20.52°± 0.2°, 22.36°± 0.2, 23.67°± 0.2°, 25.89°± 0.2°, 26.39°± 0.2°, 28.13°± 0.2°, 28.91°± 0.2°.

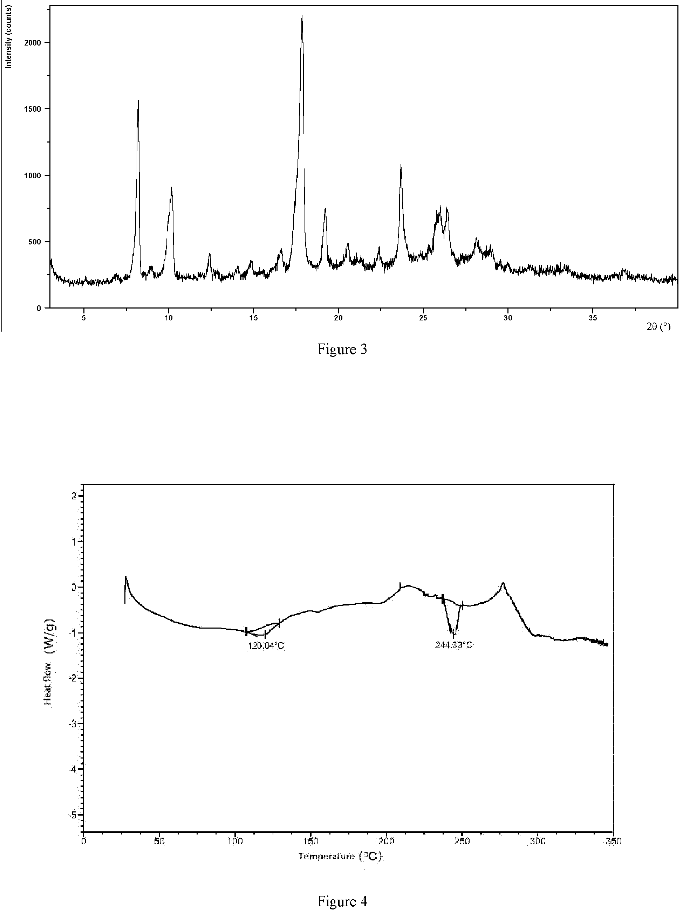

In yet other embodiments, the phosphate crystal form B of the present invention has an X-ray powder diffraction pattern substantially as shown in Figure 3.

In other embodiments, the differential scanning calorimetry diagram of the phosphate crystal form B of the present invention comprises endothermic peaks at 120.04°C ± 3°C and 244.33°C ± 3°C.

In yet other embodiments, the phosphate crystal form B of the present invention has a differential scanning calorimetry diagram substantially as shown in Figure 4.

In other embodiments, the X-ray powder diffraction pattern of the phosphate crystal form C of the present invention comprises peaks expressed as 2Θ at: 5.69°±0.2°, 10.57°±0.2°, 10.97°±0.2°, 16.76°± 0.2°, 21.40°± 0.2, 22.58°± 0.2°, 23.04°± 0.2°, 24.91°± 0.2°, 25.60°± 0.2°.

In other embodiments, the X-ray powder diffraction pattern of the phosphate crystal form C of the present invention comprises peaks expressed as 2Θ at: 5.69°± 0.2°, 6.99°± 0.2°, 10.57°±0.2°, 10.97°± 0.2°, 12.37°± 0.2°, 13.03°± 0.2°, 15.95°± 0.2°, 16.76°±0.2°, 19.43°± 0.2°, 20.23°± 0.2, 21.40°± 0.2°, 22.58°± 0.2°, 23.04°± 0.2°, 24.91°± 0.2°, 25.60°± 0.2°, 25.90°± 0.2°, 26.67°± 0.2°, 27.10°± 0.2°, 27.90°± 0.2°.

In other embodiments, the X-ray powder diffraction pattern of the phosphate crystal form C of the present invention comprises peaks expressed as 2Θ at: 5.69°± 0.2°, 6.99°± 0.2°, 10.57°± 0.2°, 10.97°± 0.2°, 12.37°± 0.2°, 13.03°± 0.2°, 14.05°± 0.2°, 15.55°± 0.2°, 15.95°± 0.2°, 16.76°± 0.2°, 17.24°± 0.2°, 18.82°± 0.2°, 19.13°± 0.2°, 19.43°± 0.2°, 20.23°± 0.2°, 20.75°± 0.2°, 21.40°± 0.2°, 22.58°± 0.2°, 23.04°± 0.2°, 24.91°± 0.2°, 25.60°± 0.2°, 25.90°± 0.2°, 26.67°± 0.2°, 27.10°± 0.2°, 27.90°± 0.2°, 28.93°± 0.2°, 29.19°± 0.2°, 32.21°± 0.2°.

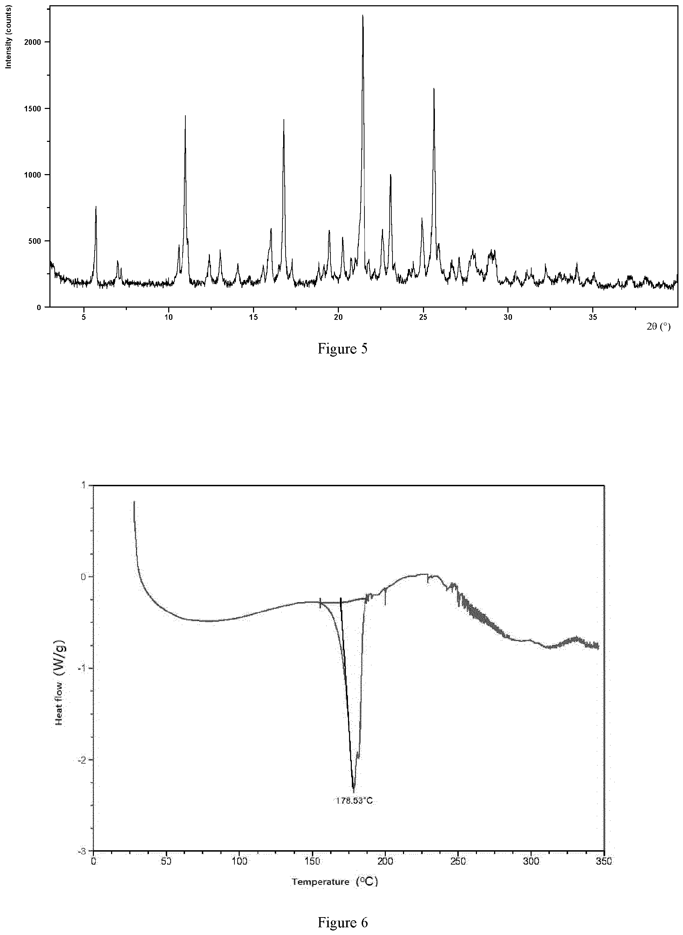

In yet other embodiments, the phosphate crystal form C of the present invention has an X-ray powder diffraction pattern substantially as shown in Figure 5.

In other embodiments, the differential scanning calorimetry diagram of the phosphate crystal form C of the present invention comprises an endothermic peak at 178.53°C ± 3°C.

In yet other embodiments, the phosphate crystal form C of the present invention has a differential scanning calorimetry diagram substantially as shown in Figure 6.

In other embodiments, the X-ray powder diffraction pattern of the phosphate crystal form G of the present invention comprises peaks expressed as 2Θ at: 6.59°±0.2°, 7.72°±0.2°, 13.23°±0.2°, 13.72°± 0.2°, 14.95°± 0.2, 18.99°± 0.2°, 24.97°± 0.2°, 25.49°± 0.2°, 26.30°± 0.2°.

In other embodiments, the X-ray powder diffraction pattern of the phosphate crystal form G of the present invention comprises peaks expressed as 2Θ at: 6.59°± 0.2°, 7.72°±0.2°, 13.23°± 0.2°, 13.72°± 0.2°, 14.95°± 0.2°, 15.49°± 0.2°, 18.07°±0.2°, 18.99°± 0.2°, 20.78°± 0.2, 21.90°± 0.2°, 23.29°± 0.2°, 24.97°± 0.2°, 25.49°± 0.2°, 26.30°± 0.2°.

In other embodiments, the X-ray powder diffraction pattern of the phosphate crystal form G of the present invention comprises peaks expressed as 2Θ at: 6.59°± 0.2°, 7.72°± 0.2°, 13.23°±0.2°, 13.72°± 0.2°, 14.95°± 0.2°, 15.49°± 0.2°, 18.07°± 0.2°, 18.99°±0.2°, 20.78°± 0.2°, 21.90°± 0.2, 23.29°± 0.2°, 24.97°± 0.2°, 25.49°± 0.2°, 26.30°± 0.2°, 26.94°± 0.2°, 27.26°± 0.2°, 27.86°± 0.2°.

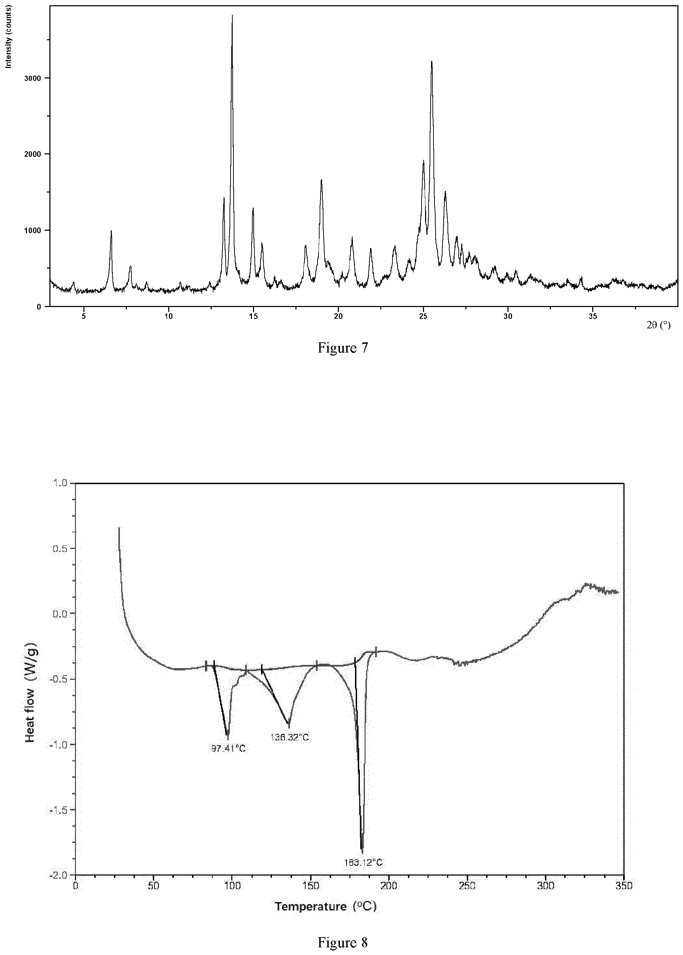

In yet other embodiments, the phosphate crystal form G of the present invention has an X-ray powder diffraction pattern substantially as shown in Figure 7.

In other embodiments, the differential scanning calorimetry diagram of the phosphate crystal form G of the present invention comprises endothermic peaks at 97.41°C ± 3°C, 136.32°C ± 3°C and 183.12°C±3°C.

In yet other embodiments, the phosphate crystal form G of the present invention has a differential scanning calorimetry diagram substantially as shown in Figure 8.

In other embodiments, the X-ray powder diffraction pattern of the phosphate crystal form H of the present invention comprises peaks expressed as 2Θ at: 7.31°±0.2°, 8.95°±0.2°, 11.81°±0.2°, 12.03°± 0.2°, 13.45°±0.2, 14.65°±0.2°, 14.74°±0.2°, 18.16°±0.2°, 18.98°±0.2°.

In other embodiments, the X-ray powder diffraction pattern of the phosphate crystal form H of the present invention comprises peaks expressed as 2Θ at: 7.31°±0.2°, 8.95°±0.2°, 11.81°±0.2°, 12.03°± 0.2°, 13.45°± 0.2°, 14.65°± 0.2°, 14.74°±0.2°, 16.21°± 0.2°, 18.16°± 0.2, 18.98°± 0.2°, 23.17°± 0.2°, 23.64°± 0.2°, 24.66°± 0.2°, 25.19°± 0.2°, 27.50°± 0.2°.

In other embodiments, the X-ray powder diffraction pattern of the phosphate crystal form H of the present invention comprises peaks expressed as 2Θ at: 7.31°± 0.2°, 8.95°± 0.2°, 11.81°±0.2°, 12.03°± 0.2°, 13.45°± 0.2°, 14.65°± 0.2°, 14.74°± 0.2°, 16.21°±0.2°, 16.48°± 0.2°, 18.16°± 0.2, 18.98°± 0.2°, 20.09°± 0.2°, 23.17°± 0.2°, 23.64°± 0.2°, 24.66°± 0.2°, 25.19°± 0.2°, 27.50°± 0.2°, 29.69°± 0.2°.

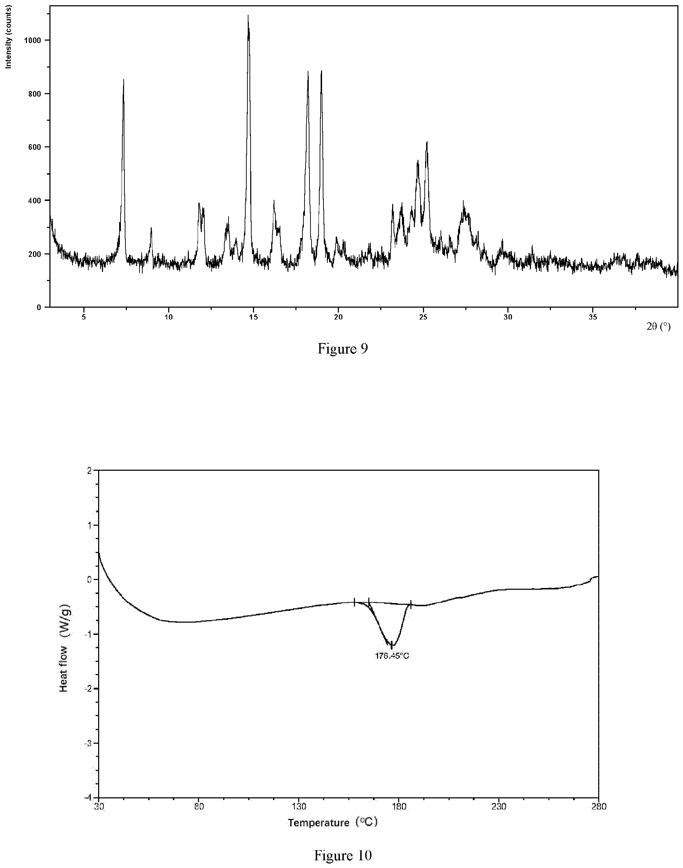

In yet other embodiments, the phosphate crystal form H of the present invention has an X-ray powder diffraction pattern substantially as shown in Figure 9.

In other embodiments, the differential scanning calorimetry diagram of the phosphate crystal form H of the present invention comprises an endothermic peak at 176.45°C ± 3°C.

In yet other embodiments, the phosphate crystal form H of the present invention has a differential scanning calorimetry diagram substantially as shown in Figure 10.

In other embodiments, the X-ray powder diffraction pattern of the phosphate crystal form A1 of the present invention comprises peaks expressed as 2Θ at: 5.67°±0.2°, 7.23°±0.2°, 11.22°±0.2°, 12.55°± 0.2°, 16.12°± 0.2°, 17.16°± 0.2°, 21.35°± 0.2°, 25.45°± 0.2°.

In other embodiments, the X-ray powder diffraction pattern of the phosphate crystal form A1 of the present invention comprises peaks expressed as 2Θ at: 5.67°± 0.2°, 7.23°±0.2°, 11.22°± 0.2°, 12.55°± 0.2°, 16.12°± 0.2°, 17.16°±0.2°, 21.35°± 0.2°, 21.96°± 0.2, 24.56°± 0.2°, 25.45°± 0.2°, 27.69°± 0.2°, 28.41°± 0.2°.

In other embodiments, the X-ray powder diffraction pattern of the phosphate crystal form A1 of the present invention comprises peaks expressed as 2Θ at: 5.67°± 0.2°, 7.23°± 0.2°, 11.22°±0.2°, 12.55°± 0.2°, 13.64°± 0.2°, 14.43°± 0.2°, 16.12°± 0.2°, 17.16°±0.2°, 19.71°± 0.2°, 20.49°± 0.2, 21.35°± 0.2°, 21.96°± 0.2°, 22.94°± 0.2°, 24.56°± 0.2°, 25.45°± 0.2°, 27.69°± 0.2°, 28.41°± 0.2°.

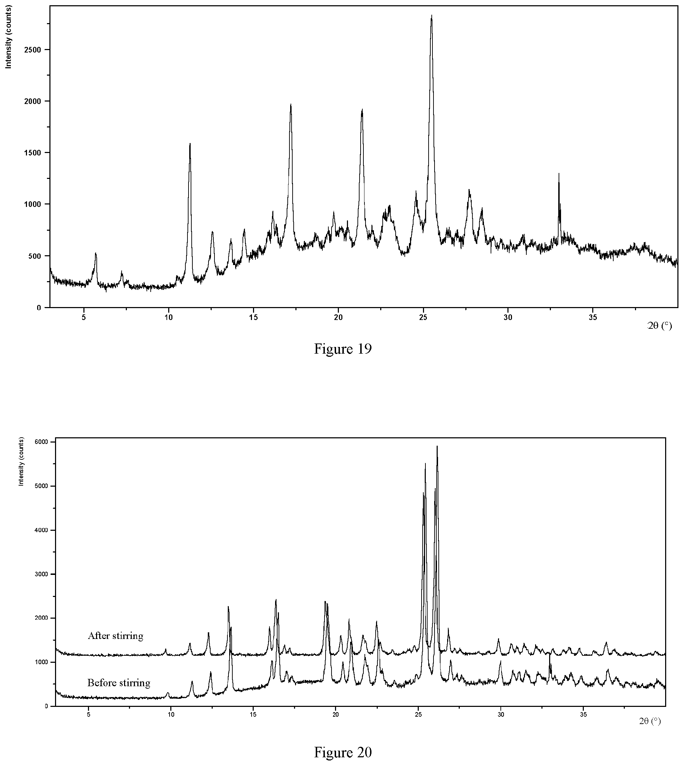

In yet other embodiments, the phosphate crystal form A1 of the present invention has an X-ray powder diffraction pattern substantially as shown in Figure 19.

In other embodiments, the salt of the compound having formula (I) of the present invention is L-tartrate, the L-tartrate is L-tartrate crystal form A, wherein the X-ray powder diffraction pattern of the L-tartrate crystal form A comprises peaks expressed as 2Θ at: 5.39°±0.2°, 6.72°± 0.2°, 14.01°± 0.2°, 14.61°± 0.2°, 15.24°±0.2°, 15.46°± 0.2°, 21.20°± 0.2, 21.75°± 0.2°, 22.81°± 0.2°, 23.85°± 0.2°, 24.55°± 0.2°.

In other embodiments, the X-ray powder diffraction pattern of the L-tartrate crystal form A of the present invention comprises peaks expressed as 2Θ at: 5.39°± 0.2°, 6.72°±0.2°, 14.01°± 0.2°, 14.61°± 0.2°, 15.24°± 0.2°, 15.46°± 0.2°, 21.20°±0.2°, 21.75°± 0.2°, 22.81°± 0.2, 23.85°± 0.2°, 24.55°± 0.2°, 27.23°± 0.2°, 27.50°± 0.2°.

In other embodiments, the X-ray powder diffraction pattern of the L-tartrate crystal form A of the present invention comprises peaks expressed as 2Θ at: 5.39°± 0.2°, 6.72°± 0.2°, 14.01°± 0.2°, 14.61°± 0.2°, 15.24°± 0.2°, 15.46°± 0.2°, 17.11°± 0.2°, 18.46°± 0.2°, 18.66°± 0.2°, 19.21°± 0.2°, 20.33°± 0.2°, 21.20°± 0.2°, 21.75°± 0.2°, 22.81°± 0.2°, 23.85°± 0.2°, 24.55°± 0.2°, 25.65°± 0.2°, 26.70°± 0.2°, 27.23°± 0.2°, 27.50°± 0.2°, 29.34°± 0.2°.

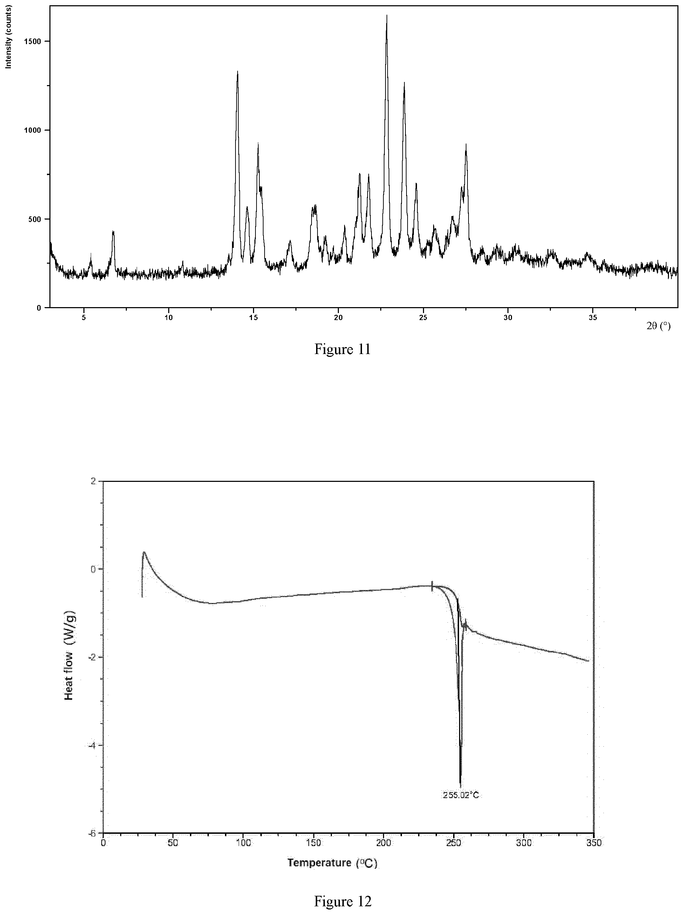

In yet other embodiments, the L-tartrate crystal form A of the present invention has an X-ray powder diffraction pattern substantially as shown in Figure 11.

In other embodiments, the differential scanning calorimetry diagram of the L-tartrate crystal form A of the present invention comprises an endothermic peak at 255.02°C ± 3°C.

In yet other embodiments, the L-tartrate crystal form A of the present invention has a differential scanning calorimetry diagram substantially as shown in Figure 12.

In some embodiments, the salt of the compound having formula (I) of the present invention is benzenesulfonate, the benzenesulfonate is benzenesulfonate crystal form A, wherein the X-ray powder diffraction pattern of the benzenesulfonate crystal form A comprises peaks expressed as 2Θ at: 5.69°± 0.2°, 15.14°±0.2°, 15.58°± 0.2°, 17.05°± 0.2, 18.63°± 0.2°, 20.36°± 0.2°, 20.98°± 0.2°, 26.36°± 0.2°.

In other embodiments, the X-ray powder diffraction pattern of the benzenesulfonate crystal form A of the present invention comprises peaks expressed as 2Θ at: 5.69°± 0.2°, 15.14°±0.2°, 15.58°±0.2°, 17.05°±0.2°, 17.82°± 0.2°, 18.63°± 0.2, 20.36°± 0.2°, 20.98°± 0.2°, 26.36°± 0.2°, 27.81°± 0.2°.

In other embodiments, the X-ray powder diffraction pattern of the benzenesulfonate crystal form A of the present invention comprises peaks expressed as 2Θ at: 5.69°± 0.2°, 10.45°± 0.2°, 11.37°± 0.2°, 11.72°± 0.2°, 14.18°± 0.2°, 15.14°± 0.2°, 15.58°± 0.2°, 17.05°± 0.2°, 17.82°± 0.2°, 18.63°± 0.2°, 19.26°± 0.2°, 19.67°± 0.2°, 20.36°± 0.2°, 20.98°± 0.2°, 23.21°± 0.2°, 24.23°± 0.2°, 24.54°± 0.2°, 25.00°± 0.2°, 25.74°± 0.2°, 26.36°± 0.2°, 27.41°± 0.2°, 27.81°± 0.2°, 28.52°± 0.2°, 29.65°± 0.2°, 30.53°± 0.2°, 31.09°± 0.2°, 31.55°± 0.2°, 32.19°± 0.2°, 33.43°± 0.2°, 38.98°± 0.2°.

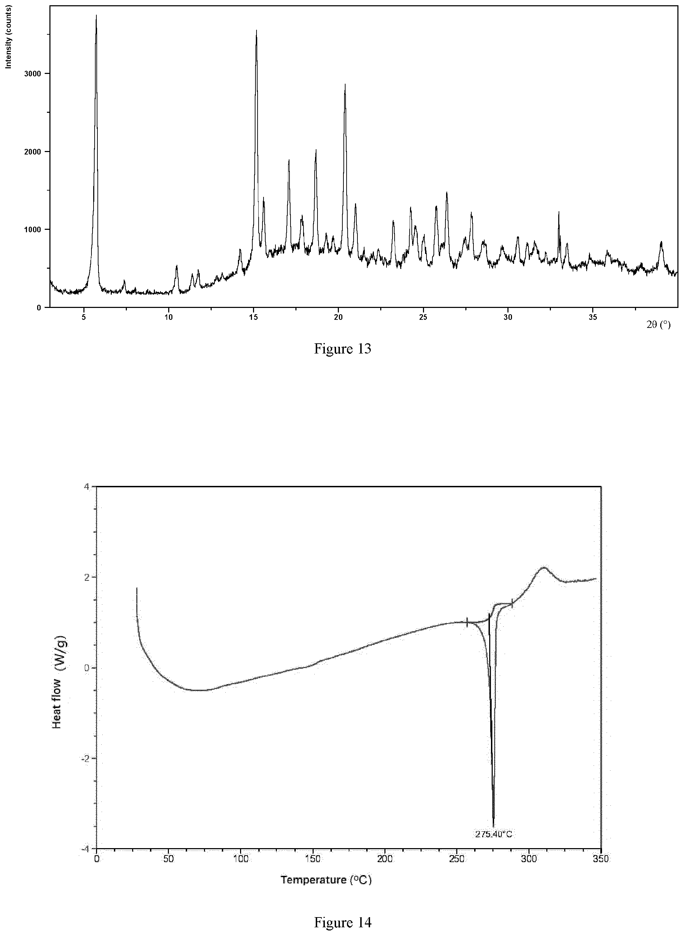

In yet other embodiments, the benzenesulfonate crystal form A of the present invention has an X-ray powder diffraction pattern substantially as shown in Figure 13.

In other embodiments, the differential scanning calorimetry diagram of the benzenesulfonate crystal form A of the present invention comprises an endothermic peak at 275.40°C ± 3°C.

In yet other embodiments, the benzenesulfonate crystal form A of the present invention has a differential scanning calorimetry diagram substantially as shown in Figure 14.

In some embodiments, the salt of the compound having formula (I) of the present invention is p-toluenesulfonate, the p-toluenesulfonate is p-toluenesulfonate crystal form A, wherein the X-ray powder diffraction pattern of the p-toluenesulfonate crystal form A comprises peaks expressed as 2Θ at: 4.68°± 0.2°, 9.30°±0.2°, 13.94°± 0.2°, 17.70°± 0.2, 18.26°± 0.2°, 19.55°± 0.2°, 19.88°± 0.2°, 24.33°± 0.2°.

In other embodiments, the X-ray powder diffraction pattern of the p-toluenesulfonate crystal form A of the present invention comprises peaks expressed as 2Θ at: 4.68°± 0.2°, 9.30°±0.2°, 11.60°±0.2°, 13.74°±0.2°, 13.94°± 0.2°, 16.73°± 0.2, 17.70°± 0.2°, 18.26°± 0.2°, 19.55°± 0.2°, 19.88°± 0.2°, 24.33°± 0.2°.

In other embodiments, the X-ray powder diffraction pattern of the p-toluenesulfonate crystal form A of the present invention comprises peaks expressed as 2Θ at: 4.68°± 0.2°, 9.30°±0.2°, 11.60°± 0.2°, 13.74°± 0.2°, 13.94°± 0.2°, 15.22°± 0.2°, 15.57°±0.2°, 16.37°± 0.2°, 16.73°± 0.2, 17.70°± 0.2°, 18.26°± 0.2°, 19.55°± 0.2°, 19.88°± 0.2°, 24.33°± 0.2°.

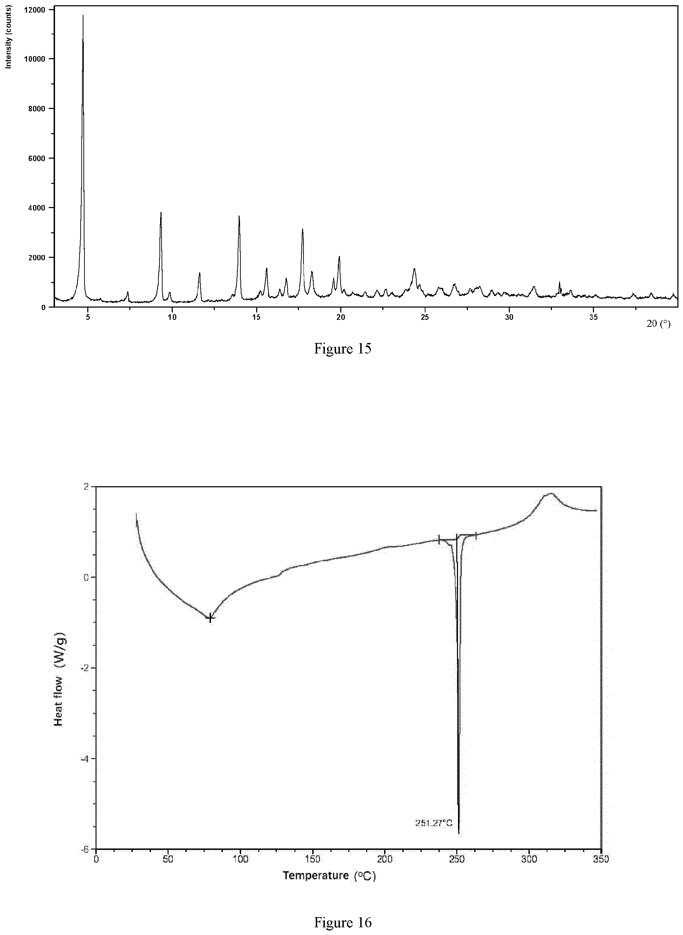

In yet other embodiments, the p-toluenesulfonate crystal form A of the present invention has an X-ray powder diffraction pattern substantially as shown in Figure 15.

In other embodiments, the differential scanning calorimetry diagram of the p-toluenesulfonate crystal form A of the present invention comprises an endothermic peak at 251.27°C ± 3°C.

In yet other embodiments, the p-toluenesulfonate crystal form A of the present invention has a differential scanning calorimetry diagram substantially as shown in Figure 16.

In some embodiments, the salt of the compound having formula (I) of the present invention is sulfate, the sulfate is sulfate crystal form A, wherein the X-ray powder diffraction pattern of the sulfate crystal form A comprises peaks expressed as 2Θ at: 11.29°± 0.2°, 13.77°±0.2°, 14.64°± 0.2°, 16.15°± 0.2, 18.23°± 0.2°, 23.90°± 0.2°, 24.96°± 0.2°, 25.84°± 0.2°.

In other embodiments, the X-ray powder diffraction pattern of the sulfate crystal form A of the present invention comprises peaks expressed as 2Θ at: 11.29°± 0.2°, 13.77°±0.2°, 14.64°±0.2°, 16.15°±0.2°, 18.23°± 0.2°, 23.90°± 0.2, 24.96°± 0.2°, 25.84°± 0.2°, 27.65°± 0.2°, 29.39°± 0.2°, 31.80°± 0.2°.

In other embodiments, the X-ray powder diffraction pattern of the sulfate crystal form A of the present invention comprises peaks expressed as 2Θ at: 11.29°± 0.2°, 13.77°±0.2°, 14.64°± 0.2°, 16.15°± 0.2°, 18.23°± 0.2°, 19.82°± 0.2°, 20.67°±0.2°, 22.14°± 0.2°, 23.90°± 0.2, 24.96°± 0.2°, 25.84°± 0.2°, 27.65°± 0.2°, 29.39°± 0.2°, 31.80°± 0.2°.

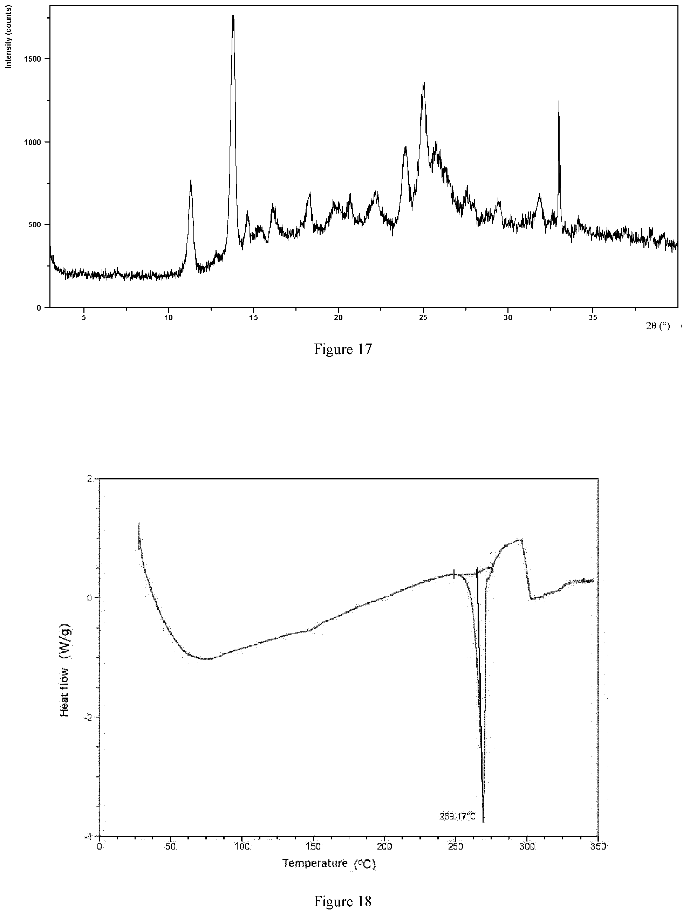

In yet other embodiments, the sulfate crystal form A of the present invention has an X-ray powder diffraction pattern substantially as shown in Figure 17.

In other embodiments, the differential scanning calorimetry diagram of the sulfate crystal form A of the present invention comprises an endothermic peak at 269.17°C ± 3°C.

In yet other embodiments, the sulfate crystal form A of the present invention has a differential scanning calorimetry diagram substantially as shown in Figure 18.

In another aspect, the present invention relates to a pharmaceutical composition comprising any one of the salts of the present invention or any one of the crystal forms of the salts, and a pharmaceutically acceptable carrier, excipient, diluent, adjuvant or a combination thereof.

In one aspect, the present invention relates to use of the salt or its crystal form or the pharmaceutical composition in the manufacture of a medicament for preventing, treating or alleviating central nervous system dysfunction.

In some embodiments, the central nervous system dysfunctions described herein include, but are not limited to, depression, anxiety disorder, mania, schizophrenia, bipolar disorder, sleep disorder, obsession and behavior disorder, panic disorder, post-traumatic stress disorder (PTSD), movement disorders, sexual dysfunction, musculoskeletal pain disorder, cognitive impairment, memory impairment, Parkinson's disease, Huntington's disease, phobias, substance abuse or addiction, drug addiction withdrawal symptoms or premenstrual stress syndrome.

In another aspect, the present invention relates to use of the salt or its crystal form or the pharmaceutical composition in the manufacture of a medicament, wherein the medicament is used as a selective 5-hydroxytryptamine reuptake inhibitor and/or 5-HT_{1A} receptor agonist.

In one aspect, the present invention relates to the salt or its crystal form or the pharmaceutical composition for use in preventing, treating or alleviating central nervous system dysfunction.

In some embodiments, the central nervous system dysfunctions described herein include, but are not limited to, depression, anxiety disorder, mania, schizophrenia, bipolar disorder, sleep disorder, obsession and behavior disorder, panic disorder, post-traumatic stress disorder (PTSD), movement disorders, sexual dysfunction, musculoskeletal pain disorder, cognitive impairment, memory impairment, Parkinson's disease, Huntington's disease, phobias, substance abuse or addiction, drug addiction withdrawal symptoms or premenstrual stress syndrome.

In another aspect, the present invention relates to the salt or its crystal form or the pharmaceutical composition for use as a selective 5-hydroxytryptamine reuptake inhibitor and/or 5-HT_{1A} receptor agonist.

In one aspect, the present invention relates to a method of preventing, treating or alleviating central nervous system dysfunction in a subject, comprising contacting the subject with a therapeutically effective amount of the salt or its crystal form or the pharmaceutical composition.

In some embodiments, the central nervous system dysfunctions described herein include, but are not limited to, depression, anxiety disorder, mania, schizophrenia, bipolar disorder, sleep disorder, obsession and behavior disorder, panic disorder, post-traumatic stress disorder (PTSD), movement disorders, sexual dysfunction, musculoskeletal pain disorder, cognitive impairment, memory impairment, Parkinson's disease, Huntington's disease, phobias, substance abuse or addiction, drug addiction withdrawal symptoms or premenstrual stress syndrome.

In one aspect, the present invention relates to a method of selectively inhibiting 5-hydroxytryptamine reuptake and/or agonizing 5-HT_{1A} receptor, comprising using the salt or its crystal form or the pharmaceutical composition.

In another aspect, the present invention also relates to a method for preparing the salt and/or crystal form of the compound having formula (I).

The solvent used in the preparation method of the salt and/or crystal form of the present invention is not particularly restricted, and any solvent which dissolves the starting material to a degree and does not affect its properties is contained in the present invention. Additionally, many similar modifications in the art, equivalent replacements, or solvent, solvent composition and the solvent composition with different proportions which are equivalent to those described in the invention, all are deemed to be included in the present invention. The present invention gives the preferred solvent for each reaction step.

The preparation of the salt or crystal form of the present invention will be described in detail in the examples section. Meanwhile, the present invention provides property testing experiments of the salts or crystal forms, such as pharmacokinetic experiments, solubility experiments, stability experiments and/or hygroscopicity experiments. It is found through experiments that the phosphate crystal form A of the present invention has unexpected technical advantages. The phosphate crystal form A of the present invention has higher blood concentration and exposure in dogs, thereby having better pharmacokinetic properties; meanwhile, the phosphate crystal form A of the present invention has particularly good stability. Under high temperature, high humidity or light conditions, the purity basically does not change, and the crystal structure does not change; in addition, the phosphate crystal form A is stirred in water at room temperature or under heating conditions, and the crystal structure does not change; and the phosphate crystal form A of the present invention has low hygroscopicity and is not easily affected by high humidity to deliquescence. It can be seen that the crystal form has stable properties, is more suitable for technological production, and is easy to store.

Therefore, the phosphate crystal form A of the present invention has good biological activity and stability, and at the same time has low hygroscopicity, and is more suitable for pharmaceutical use.

### DEFINITIONS AND GENERAL TERMINOLOGY

Unless otherwise indicated, all technical and scientific terms used in the present invention have the same meaning as commonly understood by one of ordinary skill in the art to which this invention pertains. All patents and publications referred to herein are incorporated by reference in their entirety. Although any methods and materials similar or identical to those described herein may be used in the practice or testing of the invention, but the methods, apparatus and materials described in the invention are preferred.

"Crystal form" or "crystalline form" refers to a solid having a highly regular chemical structure, including, but not limiting to, mono- or multi-component crystals, and/or polymorphs of compounds, solvates, hydrates, clathrates, eutectic, salt, solvates of salt, hydrates of salt. The crystalline form of the material can be obtained by a number of methods known in the field. Such methods include, but are not limited to, melt crystallization, melt cooling, solvent crystallization, crystallization in defined space, for example, in nanopores or capillaries, on a surface or template, for example, on a polymer, in the presence of additives such as co-crystallized antimolecules, removing solvent, dehydration, rapid evaporation, rapid cooling, slow cooling, vapor diffusion, sublimation, reaction crystallization, anti-solvent addition, grinding and solvent drop milling, and the like.

"Solvent" refers to a substance (typically a liquid) that is capable of completely or partially dissolving another substance (typically a solid). Solvents for use in the practice of this invention include, but are not limited to, water, acetic acid, acetone, acetonitrile, benzene, chloroform, carbon tetrachloride, dichloromethane, dimethylsulfoxide, 1,4-dioxane, ethanol, ethyl acetate, butanol, t-butanol, N, A-dimethylacetamide, *N,* A-dimethylformamide, formamide, formic acid, heptane, hexane, isopropanol, methanol, methyl ethyl ketone, mesitylene, nitromethane, ethylene glycol, propanol, pyridine, tetrahydrofuran, toluene, xylene, mixtures thereof, and the like.

"Solvate" refers to a compound having a solvent on a surface, in a lattice or both on a surface or in a lattice. The solvent can be water, acetic acid, acetone, acetonitrile, benzene, chloroform, carbon tetrachloride, dichloromethane, dimethylsulfoxide, 1,4-dioxane, ethanol, ethyl acetate, butanol, t-butanol, N, A-dimethylacetamide, N, A-dimethylformamide, formamide, formic acid, heptane, hexane, isopropanol, methanol, methyl ethyl ketone, methyl pyrrolidone, mesitylene, nitromethane, ethylene glycol, propanol, pyridine, tetrahydrofuran, toluene, xylene, mixtures thereof, and the like. A specific example of the solvate is a hydrate in which the solvent on the surface, in the lattice or both on the surface and in the lattice is water. On the surface, in the lattice or both on the surface and in the lattice of the substance, the hydrate may or may not have any solvent other than water.

Crystal form can be identified by a variety of technical means, such as X-ray powder diffraction (XRPD), infrared absorption spectroscopy (IR), melting point method, differential scanning calorimetry (DSC), thermogravimetric analysis (TGA), Nuclear magnetic resonance, Raman spectroscopy, X-ray single crystal diffraction, dissolution calorimetry, scanning electron microscopy (SEM), quantitative analysis, solubility and dissolution rate.

X-ray powder diffraction (XRPD) can detect changes in crystal form, crystallinity, crystal state and other information, is a common means for identifying crystal form. The peak position of the XRPD pattern primarily depends on the structure of the crystal form and is relatively insensitive to the experimental details, and its relative peak height depends on many factors associated with sample preparation and instrument geometry. Thus, in some embodiments, the crystalline form of the present invention is characterized by an XRPD pattern having certain peak positions, which is substantially as shown in the XRPD diagram provided in the drawings of the present invention. At the same time, the 2Θ of the XRPD pattern can be measured with an experimental error. The measurement of 2Θ of the XRPD pattern may be slightly different between the different instruments and between the different samples. Therefore, the value of 2Θ cannot be regarded as absolute. According to the condition of the instrument used in this test, the diffraction peak has an error tolerance of ±0.2°.

Differential Scanning Calorimetry (DSC) is a technique of measuring the energy difference between a sample and an inert reference (commonly used α-Al₂O₃) with temperature by continuously heating or cooling under program control. The endothermic peak heights of the DSC diagram depend on many factors associated with sample preparation and instrument geometry, while the peak position is relatively insensitive to experimental details. Thus, in some embodiments, the crystal form of the present invention is characterized by a DSC diagram having certain peak positions, which is substantially as shown in the DSC diagram provided in the drawings of the present invention. At the same time, the DSC diagram can be measured with an experimental error. The peak position and peak value of DSCdiagram may be slightly different between the different instruments and between the different samples. Therefore, the peak position or the peak value of the DSC endothermic peak cannot be regarded as absolute. According to the condition of the instrument used in this test, the endothermic peak has an error tolerance of ± 3°.

Thermogravimetric analysis (TGA) is a technique for measuring the quality of a substance with temperature under the control of a program. It is suitable for examining the process of the solvent loss or the samples sublimation and decomposition. It can be presumed that the crystal contains crystal water or crystallization solvent. The change in mass by the TGA curve shown depends on a number of factors containing the sample preparation and the instrument. The change in mass by the TGA test may be slightly different between the different instruments and between the different samples. According to the condition of the instrument used in this test, there is an error tolerance of ± 0.1% for the change in mass.

In the context of the present invention, the 2Θ values in the X-ray powder diffraction pattern are in degrees (°).

The term "substantially as shown in the figure" refers to at least 50%, or at least 60%, or at least 70%, or at least 80%, or at least 90%, or at least 95%, or at least 99% of the peaks are shown in the X-ray powder diffraction pattern or DSC pattern or Raman spectra pattern or infrared spectra pattern.

The "peak" refers to a feature that a person skilled in the art can recognize without belonging to background noise when referring to a spectrum or/and data that appears in the figure.

The present invention relates to the salts of the compounds having formula (I) and/or crystal forms thereof, which exist in substantially pure crystalline form.

"Substantially pure" means that a crystalline form is substantially free of another or more crystalline forms, that means the purity of the crystalline form is at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or crystal form containing other crystal form. The percentage of the other crystals in the total volume or total weight of the crystal form is less than 20%, or less than 10%, or less than 5%, or less than 3%, or less than 1%, or less than 0.5%, or less than 0.1%, or less than 0.01%.

"Substantially free" means that the percentage of one or more other crystalline forms in the total volume or total weight of the crystalline form is less than 20%, or less than 10%, or less than 5%, or less than 4%, or less than 3%, or less than 2%, or less than 1%, or less than 0.5%, or less than 0.1%, or less than 0.01%.

The "relative intensity" (or "relative peak height") in the XRPD pattern means the ratio of the intensity of the other peaks to the intensity of the first strong peak when the intensity of the first strong peak in all the diffraction peaks of the X-ray powder diffraction pattern (XRPD) is 100%.

In the context of the present invention, when used or whether or not used the word, such as "about", it means that within a given value or range of 10% or less, appropriately within 5%, especially within 1%. Or, for those of ordinary skill in the art, the term "about" means within an acceptable standard error range of the mean value. When a number with an N value is made public, any number within N +/- 1%, N +/- 2%, N +/- 3%, N +/- 5%, N +/- 7%, N +/- 8%, or N +/- 10% will be opened clearly, wherein "+/-" means plus or minus.

In the present invention, "room temperature" refers to a temperature from about 10 °C to about 40 °C. In some embodiments, "room temperature" refers to a temperature from about 20 °C to about 30 °C; in other embodiments, "room temperature" refers to 20 °C, 22.5 °C, 25 °C, 27.5 °C, and the like.

### PHARMACEUTICAL COMPOSITIONS, FORMULATIONS, ADMINISTRATION AND USES OF THE SALTS OR THEIR CRYSTAL FORMS OF THE PRESENT INVENTION

The characteristics of the pharmaceutical composition of the present invention include the salt of the compound having formula (I) and/or its crystal form and a pharmaceutically acceptable carrier, adjuvant, or excipient. The amount of the salt of the compound or its crystal form in the pharmaceutical composition of the present invention is effective to detectably treat or reduce central nervous system dysfunction in a patient. The pharmaceutical compositions of the present invention may also optionally contain other therapeutic and/or prophylactic ingredients.

Suitable carriers, adjuvants and excipients are well known to those skilled in the art and are described in detail in, for example, Ansel H. C. et al., Ansel's Pharmaceutical Dosage Forms and Drug Delivery Systems (2004) Lippincott, Williams & Wilkins, Philadelphia; Gennaro A. R. et al., Remington: The Science and Practice of Pharmacy (2000) Lippincott, Williams & Wilkins, Philadelphia; and Rowe R. C., Handbook of Pharmaceutical Excipients (2005) Pharmaceutical Press, Chicago.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York, the contents of each of which are incorporated by reference herein, are disclosed various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, carriers in the known technology and their uses are contemplated to be within the scope of this invention.

The pharmaceutical compositions of the invention are prepared using techniques and methods known to those skilled in the art. Some of the methods commonly used in the art are described in Remington's Pharmaceutical Sciences (Mack Publishing Company).

Another aspect of the present invention is related to a method for preparing a pharmaceutical composition, the pharmaceutical composition contains a salt of the compound of the present invention or a crystal form thereof and a pharmaceutically acceptable excipient, carrier, adjuvant, vehicle or a combination thereof; the method comprises mixing various ingredients. The pharmaceutical composition comprising a salt of a compound of the present invention, or a crystal form thereof, can be prepared for example at environment temperature and under barometric pressure.

The compound of the invention or salt thereof will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams, and gels.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

In one embodiment, the therapeutic methods disclosed herein comprise administrating to a patient in need of the treatment a safe and effective amount of the salt of the compound of the invention or the pharmaceutical composition containing the salt of the compound of the invention. Each example disclosed herein comprises the method of treating the above disorders or diseases comprising administrating to a patient in need of the treatment a safe and effective amount of the salt of the compound of the invention or the pharmaceutical composition containing the salt of the compound of the invention.

In one embodiment, the salt of the compound of the invention or the pharmaceutical composition containing the salt of the compound of the invention thereof may be administered by any suitable route of administration, including both systemic administration and topical administration. Systemic administration includes oral administration, parenteral administration, transdermal administration and rectal administration. Typical parenteral administration refers to administration by injection or infusion, including intravenous, intramuscular, and subcutaneous injection or infusion. Topical administration includes application to the skin as well as intraocular, otic, intravaginal, inhaled and intranasal administration. In one embodiment, the salt of the compound of the invention or the pharmaceutical composition containing the salt of the compound of the invention may be administered orally. In another embodiment, the salt of the compound of the invention or the pharmaceutical composition containing the salt of the compound of the invention may be administered by inhalation. In a further embodiment, the salt of the compound of the invention or the pharmaceutical composition containing the salt of the compound of the invention may be administered intranasally.

In one embodiment, the salt of the compound of the invention or the pharmaceutical composition containing the salt of the compound of the invention may be administered once or according to a dosing regimen wherein a number of doses are administered at varying intervals of time for a given period of time. For example, doses may be administered once, twice, three, or four times per day. In one embodiment, a dose is administered once per day. In a further embodiment, a dose is administered twice per day. Doses may be administered until the desired therapeutic effect is achieved or indefinitely to maintain the desired therapeutic effect. Suitable dosing regimens for the salt of the compound of the invention or the pharmaceutical composition containing the salt of the compound of the invention depend on the pharmacokinetic properties of that compound, such as absorption, distribution, and half-life, which can be determined by the skilled artisan. In addition, suitable dosing regimens, including the duration such regimens are administered, for the salt of the compound of the invention or the pharmaceutical composition containing the salt of the compound of the invention depend on the disorder being treated, the severity of the disorder being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

The salt of the compound of the present invention may be administered either simultaneously with, or before or after, one or more other therapeutic agents. The salt of the compound of the present invention may be administered separately, by the same or different route of administration, or together in the same pharmaceutical composition as the other agents. Other therapeutic agents may be amitriptyline, desipramine, mirtazapine, bupropion, reboxetine, fluoxetine, trazodone, sertraline, duloxetine, fluvoxamine, milnacipran, levomilnacipran, desvenlafaxine, vilazodone, venlafaxine, dapoxetine, nefazodone, femoxetine, clomipramine, citalopram, escitalopram, paroxetine, lithiumcarbonate, buspirone, olanzapine, quetiapine, risperidone, ziprasidone, aripiprazole, perospirone, clozapine, modafinil , mecamylamine, cabergoline, adamantane, imipramine, pramipexole, thyroxine, dextromethorphan, quinidine, naltrexone, samidorphan, buprenorphine, melatonin, alprazolam, pipamperone, vestipitant, chlordiazepoxide, perphenazine or any combination thereof, but are not limited to the above drugs.

The pharmaceutical composition or combination of the present invention can be in unit dosage of about 1-1000 mg of active ingredients for a subject of about 50-70kg. The therapeutically effective dosage of a compound, salt of a compound, pharmaceutical composition, or combination thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, e.g., mice, rats, dogs, monkeys or isolated organs, tissues and preparations thereof.

In one embodiment, a therapeutically effective dosage of the salt of the compound disclosed herein is in an amount of about 0.1 mg to about 2,000 mg per day of the compound; pharmaceutical compositions thereof should provide a dose of about 0.1 mg to about 2,000 mg of the compound. In a special embodiment, pharmaceutical dosage unit forms are prepared to provide from about 1 mg to about 2,000 mg, or about 10 mg to about 1,000 mg of the active ingredient or a combination of essential ingredients per dosage unit form.

The salts and pharmaceutical compositions of the compounds provided by the present invention can be used in the manufacture of a medicament for preventing, treating or alleviating central nervous system dysfunction in mammals, including humans, and can also be used in the manufacture of a medicament for inhibiting 5-hydroxytryptamine reuptake and/or agonizing 5-HT_{1A} receptor.

In particular, the amount of the compound in the composition of the present invention is effective to detectably selectively inhibit the reuptake of 5-hydroxytryptamine and has an agonistic effect on the 5-HT_{1A} receptor, and the salt of the compound of the present invention can be used medicines for treating human central nervous system (CNS) dysfunctions such as depression and anxiety.

The salts of the compounds disclosed herein would be useful for, but are not limited to, preventing, treating or alleviating central nervous system dysfunction by administering to the patient the salts of the compounds of the present invention or compositions disclosed herein in an effective amount. The central nervous system dysfunctions described herein in response to the regulation of 5-hydroxytryptamine receptors further include, but are not limited to, depression, anxiety disorder, mania, schizophrenia, bipolar disorder, sleep disorder, obsession and behavior disorder, panic disorder, post-traumatic stress disorder (PTSD), movement disorders, sexual dysfunction, musculoskeletal pain disorder, cognitive impairment, memory impairment, Parkinson's disease, Huntington's disease, phobias, substance abuse or addiction, drug addiction withdrawal symptoms and premenstrual stress syndrome, etc.

Besides being useful for human treatment, the salts of the compounds of the present invention and pharmaceutical compositions are also useful for veterinary treatment of mammals in companion animals, exotic animals and farm animals. In other embodiments, the animals disclosed herein include horses, dogs, and cats.

### DESCRIPTION OF THE DRAWINGS

Figure 1 is an X-ray powder diffraction (XRPD) pattern of the phosphate crystal form A of the compound of formula (I).
Figure 2 is a differential scanning calorimetry (DSC) diagram of the phosphate crystal form A of the compound of formula (I).
Figure 3 is an X-ray powder diffraction (XRPD) pattern of the phosphate crystal form B of the compound of formula (I).
Figure 4 is a differential scanning calorimetry (DSC) diagram of the phosphate crystal form B of the compound of formula (I).
Figure 5 is an X-ray powder diffraction (XRPD) pattern of the phosphate crystal form C of the compound of formula (I).
Figure 6 is a differential scanning calorimetry (DSC) diagram of the phosphate crystal form C of the compound of formula (I).
Figure 7 is an X-ray powder diffraction (XRPD) pattern of the phosphate crystal form G of the compound of formula (I).
Figure 8 is a differential scanning calorimetry (DSC) diagram of the phosphate crystal form G of the compound of formula (I).
Figure 9 is an X-ray powder diffraction (XRPD) pattern of the phosphate crystal form H of the compound of formula (I).
Figure 10 is a differential scanning calorimetry (DSC) diagram of the phosphate crystal form H of the compound of formula (I).
Figure 11 is an X-ray powder diffraction (XRPD) pattern of the L-tartrate crystal form A of the compound of formula (I).
Figure 12 is a differential scanning calorimetry (DSC) diagram of the L-tartrate crystal form A of the compound of formula (I).
Figure 13 is an X-ray powder diffraction (XRPD) pattern of the benzenesulfonate crystal form A of the compound of formula (I).
Figure 14 is a differential scanning calorimetry (DSC) diagram of the benzenesulfonate crystal form A of the compound of formula (I).
Figure 15 is an X-ray powder diffraction (XRPD) pattern of the p-toluenesulfonate crystal form A of the compound of formula (I).
Figure 16 is a differential scanning calorimetry (DSC) diagram of the p-toluenesulfonate crystal form A of the compound of formula (I).
Figure 17 is an X-ray powder diffraction (XRPD) pattern of the sulfate crystal form A of the compound of formula (I).
Figure 18 is a differential scanning calorimetry (DSC) diagram of the sulfate crystal form A of the compound of formula (I).
Figure 19 is an X-ray powder diffraction (XRPD) pattern of the phosphate crystal form A1 of the compound of formula (I).
Figure 20 is a comparison of the X-ray powder diffraction (XRPD) pattern of the phosphate crystal form A of the compound of formula (I) after stirring in water for 24 h at room temperature and the X-ray powder diffraction (XRPD) pattern before stirring.
Figure 21 is a comparison of the X-ray powder diffraction (XRPD) pattern of the phosphate crystal form B of the compound of formula (I) after stirring in water for 24 h at room temperature and the X-ray powder diffraction (XRPD) pattern before stirring.
Figure 22 is a comparison of the X-ray powder diffraction (XRPD) pattern of the phosphate crystal form C of the compound of formula (I) after stirring in water for 24 h at room temperature and the X-ray powder diffraction (XRPD) pattern before stirring.
Figure 23 is a comparison of the X-ray powder diffraction (XRPD) pattern of the phosphate crystal form G of the compound of formula (I) after stirring in water for 24 h at room temperature and the X-ray powder diffraction (XRPD) pattern before stirring.
Figure 24 is a comparison of the X-ray powder diffraction (XRPD) pattern of the phosphate crystal form H of the compound of formula (I) after stirring in water for 24 h at room temperature and the X-ray powder diffraction (XRPD) pattern before stirring.
Figure 25 is an overlay of the X-ray powder diffraction (XRPD) patterns of the phosphate crystal form A of the compound of formula (I) after being placed under high temperature (60°C), high humidity (RH 90% ± 5%) and light for 30 days.

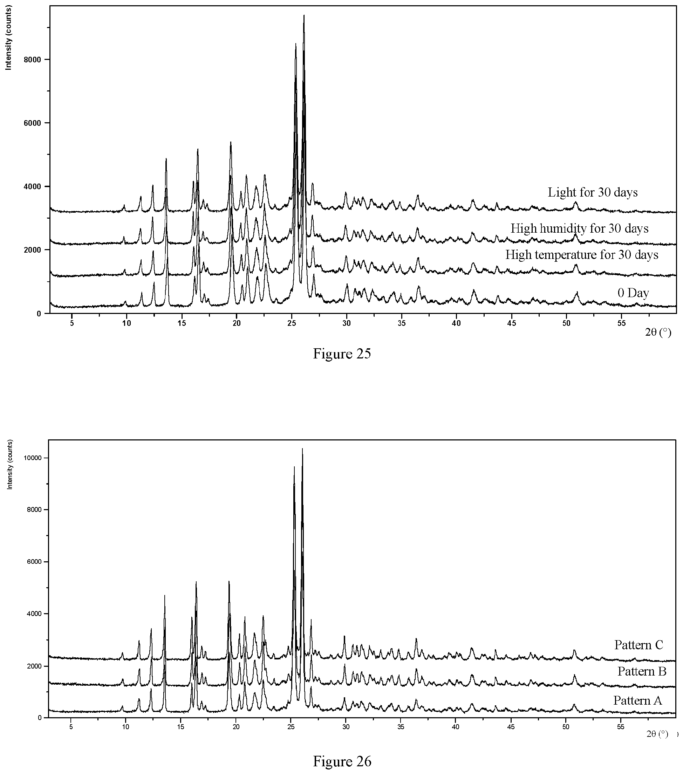

Figure 26 is an overlay of the X-ray powder diffraction (XRPD) patterns of the phosphate crystal form A of the compound of formula (I) obtained by testing under different conditions according to the method of stability test experiment C in Example 12; among them, pattern A is the XRPD pattern of phosphate crystal form A before the experiment (0 month), and pattern B is the XRPD pattern of phosphate crystal form A after being placed at 25 ± 2 °C and RH 60 ± 10% for 6 months, pattern C is the XRPD pattern of the phosphate crystal form A after being placed at 40 ± 2°C and RH 75% ± 5% for 6 months.

### DESCRIPTION OF THE PREFERRED EMBODIMENTS

The invention will now be further described by way of example without limiting the invention to the scope of the invention.

The X-ray powder diffraction analysis method used in the present invention was an Empyrean diffractometer, and an X-ray powder diffraction pattern was obtained using Cu-Ku radiation (45 KV, 40 mA). The powdery sample was prepared as a thin layer on a monocrystalline silicon sample rack or domestic silicon wafers, and placed on a rotating sample stage, analyzed with a step size of 0.0167° in the range of 3°-40° or 3°-60°. Data Collector software was used to collect data, HighScore Plus software was used to process data, Data Viewer software was used to read data. It should be noted that when using domestic silicon wafers for testing, there is a diffraction peak at 32.97° in the XRPD pattern of the tested sample, which is a background peak.

The differential scanning calorimetry (DSC) analysis method used in the present invention is a differential scanning calorimeter using a TA Q2000 module with a thermal analysis controller. Data were collected and analyzed using TA Instruments Thermal Solutions software. Approximately 1-5 mg of the sample was accurately weighed into a specially crafted aluminum crucible with a lid and analyzed from room temperature to about 300 °C-350 °C using a linear heating device at 10 °C/min. During use, the DSC chamber was purged with dry nitrogen.

### EXAMPLES

For the specific synthesis method of the compound 2-(2-(4-(3-(5-cyano-1*H*-indol-3-yl)propyl)piperazin-1-yl)pyrimidin-5-yl)-4-methylthiazol-5-carboxamide of formula (I) in free form, refer to Example 8 in the international application WO 2019062662 A1.

### Examples

### Example 1 Phosphate crystal form A1 of the present invention

### 1. Preparation of phosphate crystal form A1

The compound of formula (I) in free form (2.01 g) and DMF (40 mL) were added in a 250 mL round-bottom flask, and stirred to dissolve at room temperature. Then 1 mmol/mL phosphoric acid solution (4.8 mL) and acetone (50 mL) were added in turn. The mixture was stirred at room temperature for 24 h, and a large amount of solid was precipitated. The mixture was suction filtered, and the solid was dried *in vacuo* to obtain a light yellow solid with a yield of about 85%, which is the phosphate crystal form A1.

### 2. Characterization of phosphate crystal form A1

Analysis and identification by Empyrean X-ray powder diffraction (XRPD): Cu-Kα radiation was used and the pattern comprises the following characteristic peaks expressed as 2Θ at: 5.67°, 7.23°, 11.22°, 12.55°, 13.64°, 14.43°, 16.12°, 17.16°, 19.71°, 20.49°, 21.35°, 21.96°, 22.94°, 24.56°, 25.45°, 27.69°, 28.41°. There is an error tolerance of ± 0.2°.

Specifically, the XRPD pattern of the phosphate crystal form A1 prepared according to the method of Example 1 of the present invention is substantially as shown in Figure 19.

### Example 2 Phosphate crystal form A of the present invention

### 1. Preparation of phosphate crystal form A

The compound of formula (I) in free form (2.01 g) and DMF (40 mL) were added in a 250 mL round-bottom flask, and stirred to dissolve at room temperature. Then 1 mmol/mL phosphoric acid solution (4.8 mL) and acetone (50 mL) were added in turn. The mixture was stirred at room temperature for 24 h, and a large amount of solid was precipitated. The mixture was suction filtered, and purified water (50 mL) was added to the dried solid. The resulting mixture was heated to 80 °C and stirred for 8 h, suction filtered, and dried *in vacuo* to obtain a pale yellow solid with a yield of about 85%, which is phosphate crystal form A.

### 2. Characterization of phosphate crystal form A

(1) Analysis and identification by Empyrean X-ray powder diffraction (XRPD): Cu-Kα radiation was used and the pattern comprises the following characteristic peaks expressed as 2Θ at: 9.65°, 11.14°, 12.25°, 13.50°, 15.98°, 16.28°, 16.38°, 16.86°, 17.20°, 19.36°, 20.31°, 20.79°, 20.93°, 21.68°, 22.52°, 24.73°, 25.26°, 25.98°, 26.82°, 29.82°, 30.58°, 31.43°, 32.14°, 36.35°. There is an error tolerance of ± 0.2°.

Specifically, the XRPD pattern of the phosphate crystal form A prepared according to the method of Example 2 of the present invention is substantially as shown in Figure 1.

(2) Analysis and identification by TA Q2000 Differential Scanning Calorimetry: the scanning speed was 10 ° C/min and the diagram comprises an endothermic peak of 200.43 °C. There is an error tolerance of ± 3 °C.

Specifically, the DSC diagram of the phosphate crystal form A prepared according to the method of Example 2 of the present invention is substantially as shown in Figure 2.

### Example 3 Phosphate crystal form B of the present invention

### 1. Preparation of phosphate crystal form B

The phosphate crystal form A1 (500.50 mg) of the compound of formula (I) and methanol (10 mL) were sequentially added to a 25 mL round-bottom flask, and the mixture was suspended and stirred at 50 °C for 24 h. The mixture was suction filtered, and the solid was dried *in vacuo* at 50°C to obtain a light yellow solid with a yield of about 80%, which is the phosphate crystal form B.

### 2. Characterization of phosphate crystal form B

(1) Analysis and identification by Empyrean X-ray powder diffraction (XRPD): Cu-Kα radiation was used and the pattern comprises the following characteristic peaks expressed as 2Θ at: 8.17°, 10.08°, 12.38°, 14.82°, 16.55°, 17.77°, 19.18°, 20.52°, 22.36°, 23.67°, 25.89°, 26.39°, 28.13°, 28.91°. There is an error tolerance of ± 0.2°.

Specifically, the XRPD pattern of the phosphate crystal form B prepared according to the method of Example 3 of the present invention is substantially as shown in Figure 3.

(2) Analysis and identification by TA Q2000 Differential Scanning Calorimetry: the scanning speed was 10 ° C/min and the diagram comprises endothermic peaks of 120.04 °C and 244.33°C. There is an error tolerance of ± 3 °C.

Specifically, the DSC diagram of the phosphate crystal form B prepared according to the method of Example 3 of the present invention is substantially as shown in Figure 4.

### Example 4 Phosphate crystal form C of the present invention

### 1. Preparation of phosphate crystal form C

The phosphate crystal form A1 (500.50 mg) of the compound of formula (I) and n-propanol (10 mL) were sequentially added to a 25 mL round-bottom flask, and the mixture was suspended and stirred at 50 °C for 24 h. The mixture was suction filtered, and the solid was dried *in vacuo* at 50°C to obtain a light yellow solid with a yield of about 90%, which is the phosphate crystal form C.

### 2. Characterization of phosphate crystal form C

(1) Analysis and identification by Empyrean X-ray powder diffraction (XRPD): Cu-Kα radiation was used and the pattern comprises the following characteristic peaks expressed as 2Θ at: 5.69°, 6.99°, 10.57°, 10.97°, 12.37°, 13.03°, 14.05°, 15.55°, 15.95°, 16.76°, 17.24°, 18.82°, 19.13°, 19.43°, 20.23°, 20.75°, 21.40°, 22.58°, 23.04°, 24.91°, 25.60°, 25.90°, 26.67°, 27.10°, 27.90°, 28.93°, 29.19°, 32.21°. There is an error tolerance of ± 0.2°.

Specifically, the XRPD pattern of the phosphate crystal form C prepared according to the method of Example 4 of the present invention is substantially as shown in Figure 5.

(2) Analysis and identification by TA Q2000 Differential Scanning Calorimetry: the scanning speed was 10 ° C/min and the diagram comprises an endothermic peak of 178.53 °C. There is an error tolerance of ± 3 °C.

Specifically, the DSC diagram of the phosphate crystal form C prepared according to the method of Example 4 of the present invention is substantially as shown in Figure 6.

### Example 5 Phosphate crystal form G of the present invention

### 1. Preparation of phosphate crystal form G

The phosphate crystal form A1 (501 mg) of the compound of formula (I) and N-methylpyrrolidone (NMP, 7 mL) were added to a 50 mL round-bottom flask, and stirred to dissolve at 60°C. Then the anti-solvent toluene (30 mL) was added, and the mixture was stirred at room temperature for 24 h. The mixture was suction filtered, and the solid was dried *in vacuo* at 50°C to obtain a light yellow solid with a yield of about 85%, which is the phosphate crystal form G.

### 2. Characterization of phosphate crystal form G

(1) Analysis and identification by Empyrean X-ray powder diffraction (XRPD): Cu-Ku radiation was used and the pattern comprises the following characteristic peaks expressed as 2Θ at: 6.59°, 7.72°, 13.23°, 13.72°, 14.95°, 15.49°, 18.07°, 18.99°, 20.78°, 21.90°, 23.29°, 24.97°, 25.49°, 26.30°, 26.94°, 27.26°, 27.86°. There is an error tolerance of ± 0.2°.

Specifically, the XRPD pattern of the phosphate crystal form G prepared according to the method of Example 5 of the present invention is substantially as shown in Figure 7.

(2) Analysis and identification by TA Q2000 Differential Scanning Calorimetry: the scanning speed was 10 ° C/min and the diagram comprises endothermic peaks of 97.41°C, 136.32°C and 183.12°C. There is an error tolerance of ± 3 °C.

Specifically, the DSC pattern of the phosphate crystal form G prepared according to the method of Example 5 of the present invention is substantially as shown in Figure 8.

### Example 6 Phosphate crystal form H of the present invention

### 1. Preparation of phosphate crystal form H

The phosphate crystal form A1 (500.50 mg) of the compound of formula (I) and ethyl formate (10 mL) were sequentially added to a 25 mL round-bottom flask, and the mixture was suspended and stirred at 50 °C for 48 h; then the temperature was raised to 55 °C and the mixture was continuously stirred for 24 h. The mixture was suction filtered, and the solid was dried *in vacuo* at 50°C to obtain a light yellow solid with a yield of about 88%, which is the phosphate crystal form H.

### 2. Characterization of phosphate crystal form H

(1) Analysis and identification by Empyrean X-ray powder diffraction (XRPD): Cu-Kα radiation was used and the pattern comprises the following characteristic peaks expressed as 2Θ at: 7.31°, 8.95°, 11.81°, 12.03°, 13.45°, 14.65°, 14.74°, 16.21°, 16.48°, 18.16°, 18.98°, 20.09°, 23.17°, 23.64°, 24.66°, 25.19°, 27.50°, 29.69°. There is an error tolerance of ± 0.2°.

Specifically, the XRPD pattern of the phosphate crystal form H prepared according to the method of Example 6 of the present invention is substantially as shown in Figure 9.

(2) Analysis and identification by TA Q2000 Differential Scanning Calorimetry: the scanning speed was 10 ° C/min and the diagram comprises an endothermic peak of 176.45 °C. There is an error tolerance of ± 3 °C.

Specifically, the DSC diagram of the phosphate crystal form H prepared according to the method of Example 6 of the present invention is substantially as shown in Figure 10.

### Example 7 L-tartrate crystal form A of the present invention

### 1. Preparation of L-tartrate crystal form A

The compound of formula (I) in free form (501 mg) and DMSO (5 mL) were added to a 10 mL round-bottom flask, and stirred to dissolve at room temperature. Then L-tartaric acid (200 mg) and acetone (10 mL) were added to the above solution. The mixture was stirred for 24 h. The mixture was filtered, and the solid was dried *in vacuo* to obtain a light yellow solid with a yield of about 88%, which is the L-tartrate crystal form A.

### 2. Characterization of L-tartrate crystal form A

(1) Analysis and identification by Empyrean X-ray powder diffraction (XRPD): Cu-Kα radiation was used and the pattern comprises the following characteristic peaks expressed as 2Θ at: 5.39°, 6.72°, 14.01°, 14.61°, 15.24°, 15.46°, 17.11°, 18.46°, 18.66°, 19.21°, 20.33°, 21.20°, 21.75°, 22.81°, 23.85°, 24.55°, 25.65°, 26.70°, 27.23°, 27.50°, 29.34°. There is an error tolerance of ± 0.2°.

Specifically, the XRPD pattern of the L-tartrate crystal form A prepared according to the method of Example 7 of the present invention is substantially as shown in Figure 11.

(2) Analysis and identification by TA Q2000 Differential Scanning Calorimetry: the scanning speed was 10 ° C/min and the diagram comprises an endothermic peak of 255.02 °C. There is an error tolerance of ± 3 °C.

Specifically, the DSC diagram of the L-tartrate crystal form A prepared according to the method of Example 7 of the present invention is substantially as shown in Figure 12.

### Example 8 Benzenesulfonate crystal form A of the present invention

### 1. Preparation of benzenesulfonate crystal form A

The compound of formula (I) in free form (501 mg) and DMF (10 mL) were added to a 50 mL round-bottom flask, and stirred to dissolve at room temperature. Then 75% aqueous solution of benzenesulfonic acid (192 µL) and acetone (7.5 mL) were added in turn. The mixture was stirred for 24 h. The mixture was suction filtered, rinsed with acetone (2 mL), and the obtained pale yellow solid was dried *in vacuo,* the yield was about 89%, and it is benzenesulfonate crystal form A.

### 2. Characterization of benzenesulfonate crystal form A

(1) Analysis and identification by Empyrean X-ray powder diffraction (XRPD): Cu-Kα radiation was used and the pattern comprises the following characteristic peaks expressed as 2Θ at: 5.69°, 10.45°, 11.37°, 11.72°, 14.18°, 15.14°, 15.58°, 17.05°, 17.82°, 18.63°, 19.26°, 19.67°, 20.36°, 20.98°, 23.21°, 24.23°, 24.54°, 25.00°, 25.74°, 26.36°, 27.41°, 27.81°, 28.52°, 29.65°, 30.53°, 31.09°, 31.55°, 32.19°, 33.43°, 38.98°. There is an error tolerance of ± 0.2°.

Specifically, the XRPD pattern of the benzenesulfonate crystal form A prepared according to the method of Example 8 of the present invention is substantially as shown in Figure 13.

(2) Analysis and identification by TA Q2000 Differential Scanning Calorimetry: the scanning speed was 10 ° C/min and the diagram comprises an endothermic peak of 275.40 °C. There is an error tolerance of ± 3 °C.

Specifically, the DSC diagram of the benzenesulfonate crystal form A prepared according to the method of Example 8 of the present invention is substantially as shown in Figure 14.

### Example 9 p-Toluenesulfonate crystal form A of the present invention

### 1. Preparation of benzenesulfonate crystal form A

The compound of formula (I) in free form (501 mg) and DMF (10 mL) were added to a 50 mL round-bottom flask, and stirred to dissolve at room temperature. Then p-toluenesulfonic acid (240 mg) and acetone (5 mL) were added in turn. The mixture was stirred for 24 h. The mixture was suction filtered, rinsed with acetone (2 mL), and the obtained pale yellow solid was dried *in vacuo,* the yield was about 89%, and it is p-toluenesulfonate crystal form A.

### 2. Characterization of p-toluenesulfonate crystal form A

(1) Analysis and identification by Empyrean X-ray powder diffraction (XRPD): Cu-Kα radiation was used and the pattern comprises the following characteristic peaks expressed as 2Θ at: 4.68°, 9.30°, 11.60°, 13.74°, 13.94°, 15.22°, 15.57°, 16.37°, 16.73°, 17.70°, 18.26°, 19.55°, 19.88°, 24.33°. There is an error tolerance of ± 0.2°.

Specifically, the DSC diagram of the p-toluenesulfonate crystal form A prepared according to the method of Example 9 of the present invention is substantially as shown in Figure 15.

(2) Analysis and identification by TA Q2000 Differential Scanning Calorimetry: the scanning speed was 10 ° C/min and the diagram comprises an endothermic peak of 251.27 °C. There is an error tolerance of ± 3 °C.

Specifically, the DSC diagram of the p-toluenesulfonate crystal form A prepared according to the method of Example 9 of the present invention is substantially as shown in Figure 16.

### Example 10 Sulfate crystal form A of the present invention

### 1. Preparation of sulfate crystal form A

The compound of formula (I) in free form (501 mg) and DMF (10 mL) were added to a 50 mL round-bottom flask, and stirred to dissolve at room temperature. Then 1 mmol/mL dilute sulfuric acid (0.6 mL) and acetone (7 mL) were added in turn. The mixture was stirred for 24 h. The mixture was suction filtered, rinsed with acetone (2 mL), and the obtained pale yellow solid was dried *in vacuo* to obtain sulfate crystal form A with a yield of about 90%.

### 2. Characterization of sulfate crystal form A

(1) Analysis and identification by Empyrean X-ray powder diffraction (XRPD): Cu-Kα radiation was used and the pattern comprises the following characteristic peaks expressed as 2Θ at: 11.29°, 13.77°, 14.64°, 16.15°, 18.23°, 19.82°, 20.67°, 22.14°, 23.90°, 24.96°, 25.84°, 27.65°, 29.39°, 31.80°, 39.33°. There is an error tolerance of ± 0.2°.

Specifically, the XRPD pattern of the sulfate crystal form A prepared according to the method of Example 10 of the present invention is substantially as shown in Figure 17.

(2) Analysis and identification by TA Q2000 Differential Scanning Calorimetry: the scanning speed was 10 ° C/min and the diagram comprises an endothermic peak of 269.17 °C. There is an error tolerance of ± 3 °C.

Specifically, the DSC diagram of the sulfate crystal form A prepared according to the method of Example 10 of the present invention is substantially as shown in Figure 18.

### Example 11 The pharmacokinetics test of the salt of the present invention

The test samples were filled into capsules for oral administration.

Three 8-12 kg male Beagle dogs were taken orally administered capsules containing the test sample at a dose of 5 mg/kg, and blood was collected at time points of 0.25, 0.5, 1.0, 2.0, 4.0, 6.0, 8.0 and 24 h. Standard curve was plotted based on concentrations of the samples in a suitable range, the concentration of the test sample in the plasma sample was measured and quantified by AB SCIEX API4000 LC-MS / MS at MRM mode. Pharmacokinetic parameters were calculated according to drug concentration -time curve using a noncompartmental method by WinNonLin 6.3 software. Results are as shown in Table 1.

**Table 1 Pharmacokinetic data of the salt of the present invention**

| Test sample | Tₘₐₓ (h) | Cₘₐₓ (ng/ml) | AUCₗₐₛₜ (h*ng/ml) |
|---|---|---|---|
| Example 2 | 1.67 | 496 | 5240 |

### Conclusion:

It can be seen from Table 1 that the phosphate crystal form A described in Example 2 of the present invention has a large exposure in beagle dogs and has good pharmacokinetic properties.

### Example 12 The stability test of the salt of the present invention

### 1. Stability test experiment A

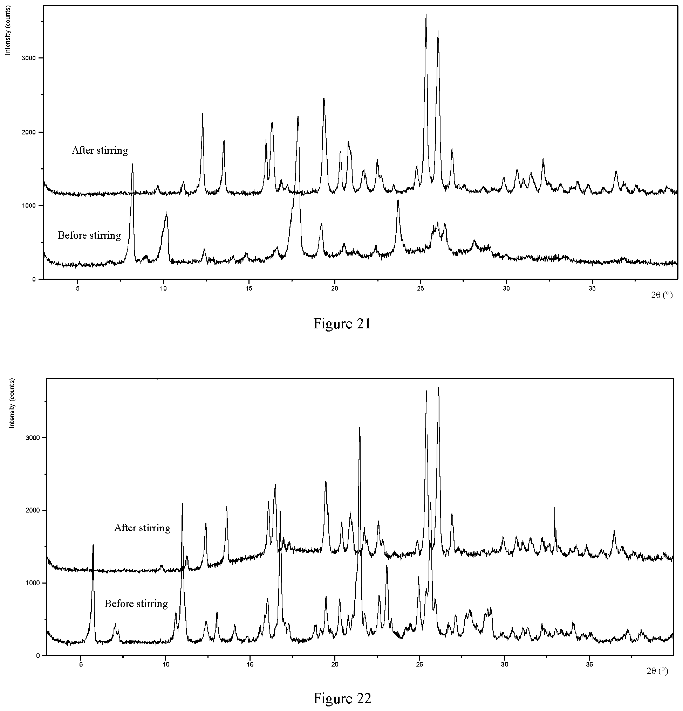

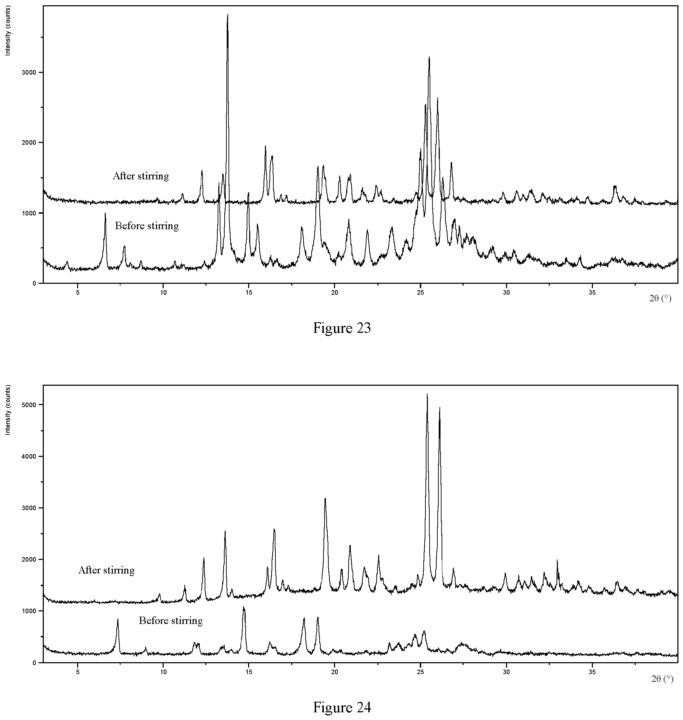

At room temperature, the test samples (50 mg) were added into EP tubes, water (1 mL) was added respectively. The mixture was suspended and stirred at room temperature for 24 h, then suction filtered, and dried for X-ray powder diffraction (XRPD) analysis and identification. Among them, the experimental results of phosphate crystal form A, phosphate crystal form B, phosphate crystal form C, phosphate crystal form G and phosphate crystal form H are shown in Figures 20-24.

It can be seen from Figures 20-24 that the phosphate crystal form A of the present invention was stirred in water at room temperature, and the crystal form did not change, while other crystal forms, such as phosphate crystal forms B, C, G and H, were also stirred in water, and the crystal form changed. That is, the phosphate crystal form A of the present invention has a stable crystal structure and is suitable for industrial production and formulation development.

### 2. Stability test experiment B

(1) High temperature test: the test samples were taken into the flat weighing bottle, spread into a thin layer of ≤ 5 mm thick, and stored for 30 days at a humidity of 75% ± 5%, 40 °C ± 2 °C and/or 60 °C ± 2 °C. On the 5th, 10th, and 30th days, samples were taken for detection according to the key stability inspection items: the color change of the sample was observed, the purity of the sample was determined by HPLC, and the crystal structure of the sample was determined by XRPD.

(2) High humidity test: the test samples were taken into the flat weighing bottle, spread into a thin layer of ≤ 5 mm thick, and stored for 30 days at 25 °C, RH 90% ± 5% and/or RH 75% ± 5%. On the 5th, 10th, and 30th days, samples were taken for detection according to the key stability inspection items: the color change of the sample was observed, the purity of the sample was determined by HPLC, and the crystal structure of the sample was determined by XRPD.

(3) Light test: the test samples were taken into the flat weighing bottle, spread into a thin layer of ≤ 5 mm thick, and placed in a light box (with an ultraviolet lamp) with an open mouth. The test samples were stored under the condition of illuminance of 4500 ± 500lx and ultraviolet light ≥ 0.7w/m² for 30 days. Samples were taken on the 5th, 10th and 30th days. The color change of the sample was observed, the purity of the sample was determined by HPLC, and the crystal structure of the sample was determined by XRPD.

The experimental results are shown in Table 2 and Figure 25.

**Table 2 The stability test results 1 of the salt of the present invention**

| No. | Condition | | High temperature (60°C, RH 75%±5%) | | | High humidity (25°C, RH 90% ± 5%) | | | Light | | |
|---|---|---|---|---|---|---|---|---|---|---|---|
| | Project | 0 day | 5 days | 10 days | 30 days | 5 days | 10 days | 30 days | 5 days | 10 days | 30 days |
| Example 2 | Outward | Light yellow solid | Light yellow solid | Light yellow solid | Light yellow solid | Light yellow solid | Light yellow solid | Light yellow solid | Yellow solid | Yellow solid | Yellow solid |
| | Purity (%) | 99.24 | 99.26 | 99.23 | 99.24 | 99.27 | 99.21 | 99.22 | 99.25 | 99.21 | 99.10 |

Experimental conclusion: It can be seen from the experimental results that under high temperature (60 °C, RH 75% ± 5%), high humidity (25 °C, RH 90% ± 5%) or light conditions, the appearance and purity of the phosphate crystal form A of the present invention have no obvious changes; at the same time, the XRPD pattern of the phosphate crystal form A is also basically unchanged. That is, the phosphate crystal form A of the present invention has good stability under various setting conditions, and is suitable for pharmaceutical use.

### 3. Stability test experiment C

1.0 g of the test samples were packaged in a single-layer PE bag and aluminum foil, and fasten with cable ties. 6 packs were prepared in total and put in a stable box (40 °C ± 2 °C, RH 75% ± 5%, or 25 °C ± 2 °C, RH 60% ± 10%). Test samples were taken at 0, 3, and 6 months for HPLC and XRPD detection, respectively. The experimental results are shown in Table 3 and Figure 26.

**Table 3 The stability test results 2 of the salt of the present invention**

| No. | Condition | | 40°C ± 2°C, RH 75% ±5% | | 25°C ± 2°C, RH 60% ±10% | |
|---|---|---|---|---|---|---|
| | Project | 0 month | 3 months | 6 months | 3 months | 6 months |
| Example 2 | Outward | Light yellow solid | Light yellow solid | Light yellow solid | Light yellow solid | Light yellow solid |
| | Purity (%) | 99.62 | 99.63 | 99.64 | 99.63 | 99.64 |

Experimental conclusion: It can be seen from the experimental results that the phosphate crystal form A of the present invention is stored for a long time under simple and conventional packaging conditions, and the purity and crystal structure have not changed, indicating the properties of the phosphate crystal form A of the present invention is stable, does not require high packaging, and is suitable for medical purposes.

### Example 13 The hygroscopicity test of the salt of the present invention

Test method:
1) A dry stoppered glass weighing bottle was placed in a suitable constant temperature desiccator (RH 80% ± 2%) at 25°C ± 1°C the day before, and accurately weighed (m1).
2) An appropriate amount of the test sample was taken and spread in the above weighing bottle. The thickness of the test sample was generally about 1 mm, and it was precisely weighed (m2).
3) The weighing bottle was opened and placed together with the bottle cap under the above constant temperature and humidity conditions for 24 hours.
4) The weighing bottle was closed with the bottle cap, and accurately weighed (m3). Calculate: weight gain % = (m3-m2) / (m2-m1) × 100%.
5) Judgment of hygroscopicity results (Chinese Pharmacopoeia 2015 Edition Appendix 9103 Guidelines for the hygroscopicity test of drugs, experimental conditions: 25°C±1°C, 80%±2% relative humidity):

| The hygroscopicity feature | | The hygroscopicity gain |
|---|---|---|
| 1 | Deliquescence | Absorb enough water to form a liquid |
| 2 | Highly hygroscopicity | Not less than 15% |
| 3 | Hygroscopicity | Less than 15% but not less than 2% |
| 4 | Slightly hygroscopicity | Less than 2% but not less than 0.2% |
| 5 | No or almost none hygroscopicity | Less than 0.2% |

Results are as shown in Table 4.

**Table 4 The hygroscopicity test results of the salts of the present invention**

| No. | The hygroscopicity gain(%) | Conclusion |
|---|---|---|
| Example 2 | 0.32 | Slightly hygroscopicity |

It can be seen from the experimental results that the phosphate crystal form A of the present invention is not easily affected by high humidity to deliquescence.

The foregoing description is merely a basic illustration of the present invention and any equivalent transformation made in accordance with the technical solution of the present invention is intended to be within the scope of the present invention.

Reference throughout this specification to "an embodiment", "some embodiments", "one embodiment", "another example", "an example", "a specific example", or "some examples" means that a particular feature, structure, material, or characteristic described in connection with the embodiment or example is included in at least one embodiment or example of the present disclosure. Thus, the appearances of the above terms throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A salt of the compound having formula (I), 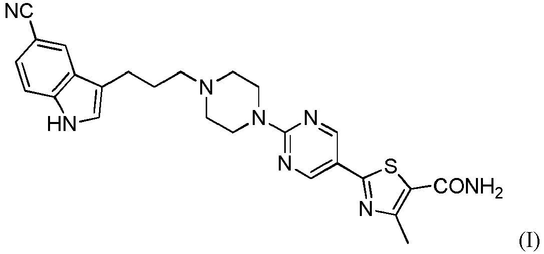 wherein, the salt is phosphate, L-tartrate, benzenesulfonate, p-toluenesulfonate or sulfate.

2. The salt of claim 1, wherein the phosphate is phosphate crystal form A, and its X-ray powder diffraction pattern comprises peaks expressed as 2Θ at: 11.14°± 0.2°, 12.25°± 0.2°, 13.50°± 0.2°, 15.98°± 0.2°, 16.38°± 0.2°, 25.26°± 0.2°, 25.98°± 0.2°, 26.82°± 0.2°; or
the phosphate is phosphate crystal form A, and its X-ray powder diffraction pattern comprises peaks expressed as 2Θ at: 11.14°± 0.2°, 12.25°± 0.2°, 13.50°± 0.2°, 15.98°± 0.2°, 16.28°± 0.2°, 16.38°± 0.2°, 25.26°± 0.2°, 25.98°± 0.2°, 26.82°± 0.2°.

3. The salt of claim 2, wherein the X-ray powder diffraction pattern of the phosphate crystal form A comprises peaks expressed as 2Θ at: 11.14°± 0.2°, 12.25°± 0.2°, 13.50°± 0.2°, 15.98°± 0.2°, 16.38°± 0.2°, 19.36°± 0.2°, 20.31°± 0.2°, 20.79°± 0.2°, 20.93°± 0.2°, 25.26°±0.2°, 25.98°± 0.2°, 26.82°± 0.2°; or
the X-ray powder diffraction pattern of the phosphate crystal form A comprises peaks expressed as 2Θ at: 11.14°± 0.2°, 12.25°± 0.2°, 13.50°± 0.2°, 15.98°± 0.2°, 16.28°± 0.2°, 16.38°± 0.2°, 19.36°± 0.2°, 20.31°± 0.2°, 20.79°± 0.2°, 20.93°± 0.2°, 25.26°± 0.2°, 25.98°± 0.2°, 26.82°± 0.2°.

4. The salt of any one of claims 2 to 3, wherein the X-ray powder diffraction pattern of the phosphate crystal form A comprises peaks expressed as 2Θ at: 9.65°± 0.2°, 11.14°± 0.2°, 12.25°± 0.2°, 13.50°± 0.2°, 15.98°± 0.2°, 16.38°± 0.2°, 16.86°±0.2°, 19.36°± 0.2°, 20.31°± 0.2°, 20.79°± 0.2°, 20.93°± 0.2°, 21.68°± 0.2°, 22.52°± 0.2°, 24.73°± 0.2°, 25.26°± 0.2°, 25.98°± 0.2°, 26.82°± 0.2°, 29.82°± 0.2°, 30.58°± 0.2°, 31.43°± 0.2°, 32.14°± 0.2°; or
the X-ray powder diffraction pattern of the phosphate crystal form A comprises peaks expressed as 2Θ at: 9.65°± 0.2°, 11.14°± 0.2°, 12.25°± 0.2°, 13.50°± 0.2°, 15.98°± 0.2°, 16.28°± 0.2°, 16.38°± 0.2°, 16.86°±0.2°, 19.36°± 0.2°, 20.31°± 0.2°, 20.79°± 0.2°, 20.93°± 0.2°, 21.68°± 0.2°, 22.52°± 0.2°, 24.73°± 0.2°, 25.26°± 0.2°, 25.98°± 0.2°, 26.82°± 0.2°, 29.82°± 0.2°, 30.58°± 0.2°, 31.43°± 0.2°, 32.14°± 0.2°.

5. The salt of any one of claims 2 to 4, wherein the phosphate crystal form A has an X-ray powder diffraction pattern substantially as shown in Figure 1.

6. The salt of any one of claims 2 to 5, wherein the differential scanning calorimetry diagram of the phosphate crystal form A comprises an endothermic peak at 200.43°C ± 3°C.

7. The salt of any one of claims 2 to 6, wherein the phosphate crystal form A has a differential scanning calorimetry diagram substantially as shown in Figure 2.

8. A pharmaceutical composition comprising the salt of any one of claims 1 to 7, and a pharmaceutically acceptable carrier, excipient, diluent, adjuvant or a combination thereof.

9. Use of the salt of any one of claims 1 to 7 or the pharmaceutical composition of claim 8 in the manufacture of a medicament for preventing, treating or alleviating central nervous system dysfunction.

10. The use of claim 9, wherein, the central nervous system dysfunction comprises depression, anxiety disorder, mania, schizophrenia, bipolar disorder, sleep disorder, obsession and behavior disorder, panic disorder, post-traumatic stress disorder, movement disorders, sexual dysfunction, musculoskeletal pain disorder, cognitive impairment, memory impairment, Parkinson's disease, Huntington's disease, phobias, substance abuse or addiction, drug addiction withdrawal symptoms or premenstrual stress syndrome.

11. The salt of any one of claims 1 to 7 or the pharmaceutical composition of claim 8 for use in preventing, treating or alleviating central nervous system dysfunction.

12. The salt or pharmaceutical composition for use according to claim 11, wherein, the central nervous system dysfunction comprises depression, anxiety disorder, mania, schizophrenia, bipolar disorder, sleep disorder, obsession and behavior disorder, panic disorder, post-traumatic stress disorder, movement disorders, sexual dysfunction, musculoskeletal pain disorder, cognitive impairment, memory impairment, Parkinson's disease, Huntington's disease, phobias, substance abuse or addiction, drug addiction withdrawal symptoms or premenstrual stress syndrome.

13. A method of preventing, treating or alleviating central nervous system dysfunction in a subject, comprising contacting the subject with a therapeutically effective amount of the salt of any one of claims 1 to 7 or the pharmaceutical composition of claim 8.

14. The method of claim 13, wherein, the central nervous system dysfunction comprises depression, anxiety disorder, mania, schizophrenia, bipolar disorder, sleep disorder, obsession and behavior disorder, panic disorder, post-traumatic stress disorder, movement disorders, sexual dysfunction, musculoskeletal pain disorder, cognitive impairment, memory impairment, Parkinson's disease, Huntington's disease, phobias, substance abuse or addiction, drug addiction withdrawal symptoms or premenstrual stress syndrome.

15. Use of the salt of any one of claims 1 to 7 or the pharmaceutical composition of claim 8 in the manufacture of a medicament, wherein the medicament is used as a selective 5-hydroxytryptamine reuptake inhibitor and/or 5-HT1A receptor agonist.

16. The salt of any one of claims 1 to 7 or the pharmaceutical composition of claim 8 for use as a selective 5-hydroxytryptamine reuptake inhibitor and/or 5-HT1_{A} receptor agonist.

17. A method of selectively inhibiting 5-hydroxytryptamine reuptake and/or agonizing 5-HT_{1A} receptor, comprising using the salt of any one of claims 1 to 7 or the pharmaceutical composition of claim 8.
